# FASCICULE DE BREVET EUROPEEN

(11) **EP 0 928 289 B1**
(45) Date de publication et mention de la délivrance du brevet: **29.09.2004**
(21) Numéro de dépôt: 98938745.1
(22) Date de dépôt: 13.07.1998
(51) Int. Cl.: C07D 233/54, A61K 7/13

(54) **NOUVELLES BASES D'OXYDATION CATIONIQUES, LEUR UTILISATION POUR LA TEINTURE D'OXYDATION DES FIBRES KERATINIQUES, COMPOSITIONS TINCTORIALES ET PROCEDES DE TEINTURE**
KATIONISCHE OXIDATIVE BASEN, IHRE VERWENDUNG ALS OXIDATIONSFÄRBEMITTEL DER KERATINFASERN, FÄRBEPRÄPARATEN UND VERFAHREN ZUM FÄRBEN
NOVEL CATIONIC OXIDATION BASES, THEIR USE FOR OXIDATION DYEING OF KERATIN FIBRES, DYEING COMPOSITIONS AND DYEING METHODS

(30) Priorité: 16.07.1997 FR 9709028
(43) Date de publication de la demande: 14.07.1999
(73) Titulaire: L'OREAL, 75008 Paris (FR)
(72) Inventeur: GENET, Alain, F-93600 Aulnay-sous-Bois (FR); LAGRANGE, Alain, F-77770 Coupvray (FR)
(74) Mandataire: Goulard, Sophie
(86) Numéro de dépôt international: PCT/FR1998/001535
(87) Numéro de publication internationale: WO 1999/003836

(56) Documents cités:
- EP-A- 0 544 400
- WO-A-95/01772
- BE-A- 616 439
- DE-B- 1 135 589
- DE-B- 1 292 784
- FR-A- 1 391 675
- TONG L K J ET AL: "The Mechanism of Dye Formation in Color Photography. VII. Intermediate Bases in the Deamination of Quinonediimines" JOURNAL OF THE AMERICAN CHEMICAL SOCIETY., vol. 82, no. 8, 25 avril 1960, pages 1988-1996, XP002060566 DC US

## Description

L'invention a pour objet de nouvelles bases d'oxydation monobenzéniques comportant au moins un groupement cationique Z, Z étant choisi parmi des chaînes aliphatiques comportant au moins un cycle insaturé quaternisé, leur utilisation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans le brevet US 5,139,532, d'utiliser certains dérivés cationiques de paraphénylènediamines, à savoir plus précisément des paraphénylènediamines dont un des groupements amino est monosubstitué par une chaîne aliphatique quaternisée, pour la teinture d'oxydation des fibres kératiniques dans des nuances intenses et plus rouges que celles obtenues habituellement en mettant en oeuvre des paraphénylènediamines classiques, c'est à dire ne portant pas de groupement cationique. Toutefois, l'utilisation des paraphénylènediamines décrites dans ce brevet antérieur ne permet pas d'obtenir une riche palette de couleurs et, de plus, les colorations obtenues ne donnent pas toujours entière satisfaction du point de vue de leur résistance vis à vis des diverses agressions que peuvent subir les cheveux (action de la lumière, de la transpiration, des shampooings, etc...).

On connaît également dans les brevets BE 616 439 et DE 1 135 589 des composés monobenzéniques comportant une fonction amine substituée par une chaîne aliphatique comportant un groupement quaternaire ; ledit groupement cationique étant relié à l'amine par un bras de liaison comprenant une fonction cétone. On connaît également dans l'article extrait du Journal of the American Chemical Society, Vol 82, N° 8, 25 avril 1960, page 1988-1996 le chlorure de 4-amino-3-méthyl-N-éthyl-N-β-(1-pyridinium)-éthyl aniline.

Or, la demanderesse vient maintenant de découvrir, de façon totalement inattendue et surprenante, que certaines nouvelles bases d'oxydation monobenzéniques de formule (I) ci-après définie, comportant au moins un groupement cationique Z, Z étant choisi parmi des chaînes aliphatiques comportant au moins un cycle insaturé quaternisé, non seulement conviennent pour une utilisation comme précurseurs de colorant d'oxydation, mais en outre qu'elles permettent d'obtenir des compositions tinctoriales conduisant à des colorations puissantes, dans une large palette de couleurs, et présentant d'excellentes propriétés de résistances aux différents traitements que peuvent subir les fibres kératiniques. Enfin, ces composés s'avèrent être aisément synthétisables.

Ces découvertes sont à la base de la présente invention.

L'invention a donc pour premier objet de nouveaux composés choisis parmi :
(a) ceux de formule (I) suivante, et leurs sels d'addition avec un acide : 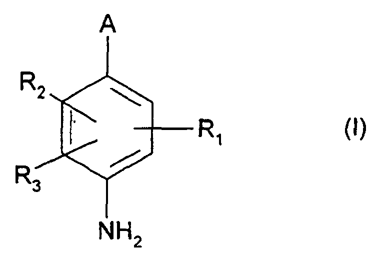 dans laquelle :
   - R₁, R₂, R₃, qui peuvent être identiques ou différents, représentent un atome d'hydrogène ; un atome d'halogène ; un groupement Z ; un radical alkyl(C₁-C₆) carbonyle ; un radical carboxy ; un radical alkyl(C₁-C₆) carboxy ; un radical carbamyle ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆,
   - R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical carboxyalkyle en C₁-C₆ ; un radical trifluoroalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆);
   - A représente un groupement -NR₄R₅ ou un radical hydroxyle ;
   - R₄ et R₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆;
   - Z est choisi parmi les groupements cationiques insaturés de formules (II) suivante : 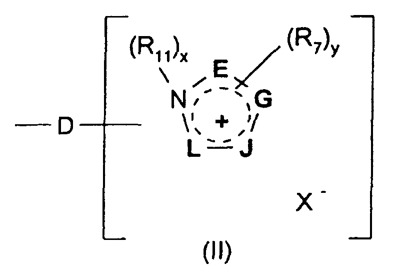
   dans lesquelles :
   - D est un bras de liaison qui représente une chaîne alkylène comportant de 1 à 14 atomes de carbone, linéaire ou ramifiée ;
   - les sommets E, G, J, L, identiques ou différents, représentent les atomes de carbone ou d'azote necessaires pour former un cycle pyrrolique, imidazolique, pyrazolique ou triazolique,
   - R₇ représente un radical alkyle en C₁-C₆, un radical monohydroxyalkyle en C₁-C₆, un radical polyhydroxyalkyle en C₂-C₆;
   - R₁₁ représente un radical alkyle en C₁-C₆ ; un radical monohydroxyalkyle en C₁-C₆ ; un radical polyhydroxyalkyle en C₂-C₆ ;
   - x et y sont des nombres entiers égaux à 0 ou 1 ; avec les conditions suivantes :
      - dans les groupements cationiques insaturés de formule (II) :
         - lorsque x = 0, le bras de liaison D est rattaché à l'atome d'azote,
         - lorsque x = 1, le bras de liaison D est rattaché à l'un des sommets E, G, J ou L,
         - y ne peut prendre la valeur 1 que :
            1) lorsque les sommets E, G, J et L représentent simultanément un atome de carbone, et que le radical R₇ est porté par l'atome d'azote du cycle insaturé ; ou bien
            2) lorsqu'au moins un des sommets E, G, J et L représente un atome d'azote sur lequel le radical R₇ est fixé ;
      X ⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
      étant entendu :
      - que le nombre de groupements cationiques insaturés Z de formule (II) est égal à 1 ;
(b) le chlorure de 1-{[5-amino-2-(2-hydroxy-éthylamino)-phénylcarbamoyl]- méthyl}-3-méthyl-3H-imidazol-1-ium et le chlorure 4-[(2,5-diamino-phénylcarbamoyl)-méthyl]-1,3-diméthyl-3H- imidazol-1-ium .

Comme indiqué précédemment, les colorations obtenues avec la composition de teinture d'oxydation conforme à l'invention sont puissantes et permettent d'atteindre une large palette de couleurs. Elles présentent de plus d'excellentes propriétés de résistance vis à vis de l'action des différents agents extérieurs (lumière, intempéries, lavage, ondulation permanente, transpiration, frottements). Ces propriétés sont particulièrement remarquables notamment en ce qui concerne la résistance des colorations obtenues vis à vis de l'action de la lumière.

Dans la formule (I) ci-dessus les radicaux alkyle et alcoxy peuvent être linéaires ou ramifiés.

Parmi les cycles des groupements insaturés Z de formule (II) ci-dessus, on peut notamment citer à titre d'exemple les cycles pyrrolique, imidazolique, pyrazolique et triazolique.

Parmi les composés de formule (I) ci-dessus, on peut notamment citer :
- le bromure de 1-[2-(4-amino-phénylamino)-éthyl]-3-méthyl-3H-imidazol-1-ium ;
- le chlorure de 1-[3-(2,5-diamino-phénoxy)-propyl]-3-méthyl-3H-imidazol-1-ium ;
- le chlorure de 3-[3-(4-amino-phénylamino)-propyl]-1-méthyl-3H-imidazol-1-ium ;
- le chlorure de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;
- le chlorure de 3-[3-(4-amino-2-fluoro-phénylamino)-propyl]-1-méthyl-3H-imidazol- 1-ium, monohydrate ;
- le chlorure de 3-[3-(4-amino-2-cyano-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 1-(5-amino-2-hydroxy-benzyl)-3-méthyl-3H-imidazol-1-ium ;
- le chlorure de 1-(5-amino-2-hydroxy-benzyl)-2-méthyl-2H-pyrazol-1-ium ;
- le chlorure de 1-[2-(2,5-diamino-phényl)-éthyl]-3-méthyl-3H-imidazol-1-ium ;
- le chlorure de 3-[2-(2,5-diamino-phényl)-éthyl]-1-méthyl-3H-imidazol-1-ium ;
- le chlorure de 1-{2-[(4-amino-phényl)-éthyl-amino]-éthyl}-3-méthyl-3H-imidazol-1-ium ;
- le dichlorure de N,N-bis-[2-(3-méthyl-3H-imidazol-1-ium)-éthyl]-4-amino-aniline ;
- le chlorure de 3-[2-(4-amino-phénylamino)-butyl]-1-méthyl-3H-imidazol-1-ium ;
- le bromure de 4-[2-(2,5-diamino-phénoxy)-éthyl]-1,3-diméthyl-3H-imidazol-1-ium ;
- le chlorure de 2-(2,5-diamino-phénoxyméthyl)-1,3-diméthyl-3H-imidazol-1-ium ;
- le chlorure de 4-[3-(4-amino-phénylamino)-propyl]-1,3-diméthyl-3H-imidazol-1-ium ;
- le chlorure de 4-[3-(4-amino-3-méthyl-phénylamino)-propyl]-1,3-diméthyl-3H-imidazol-1-ium ;
- le chlorure dé 4-{2-[2-(2-amino-5-hydroxy-phényl)-acétylamino]-éthyl}-1,3-diméthyl-3H-imidazol-1-ium ;
- le chlorure de 4-[(5-amino-2-hydroxy-benzylcarbamoyl)-méthyl]-1,3-diméthyl-3H-imidazol-1-ium ;
et leurs sels d'addition avec un acide.

Les composés de formule (I) conformes à l'invention peuvent être facilement obtenus, selon des méthodes bien connues de l'état de la technique :
- soit par réduction des composés nitrés cationiques correspondants (paranitranilines cationiques ou para-nitrophénols cationiques),
- soit par réduction des composés nitrosés cationiques correspondants (obtenus par exemple par nitrosation d'une aniline tertiaire ou d'un phénol correspondant),
- soit par réduction des composés azoïques cationiques correspondants (coupure réductrice).

Cette étape de réduction (obtention d'une amine aromatique primaire) qui confère au composé synthétisé son caractère de composé oxydable (de base d'oxydation) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quaternisation) et terminer par le "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déprotection" en milieu acide).

Lorsque la synthèse est terminée, les composés de formule (I) conformes à l'invention peuvent, le cas échéant, être récupérés par des méthodes bien connues de l'état de la technique telles que la cristallisation, la distillation.

Un autre objet de l'invention est l'utilisation des composés de formules (I) conformes à l'invention à titre de base d'oxydation pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de base d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 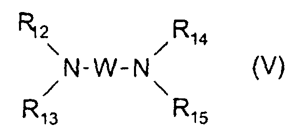 dans laquelle W est un reste propylène éventuellement substitué par un groupement hydroxyle ou un radical alkyle en C₁-C₆ ; R₁₂, R₁₃, R₁₄ et R₁₅, identiques ou différents, représentent un atome d'hydrogène, un radical alkyle en C₁-C₆ ou hydroxyalkyle en C₁-C₆.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines différentes des composés de formule (I) conformes à l'invention, les bis-phénylalkylènediamines, les para-aminophénols différents des composés de formule (I) conformes à l'invention, les ortho-aminophénols et les bases hétérocycliques.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation additionnelles et coupleurs) sont notamment choisis parmi les chlorhydrates, les bromhydrates, les sulfates, les citrates, les succinates, les tartrates, les lactates et les acétates.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES DE PREPARATION

### EXEMPLE DE PREPARATION 1 : Synthèse du monobromure, dichlorhydrate de 1-[2-(4-amino-phénylamino)-éthyl]-3-méthyl-3H-imidazol-1-ium

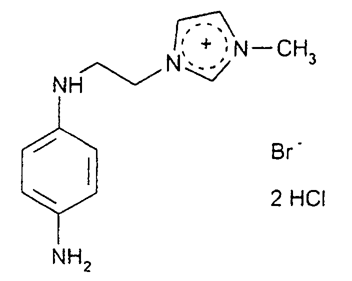

### a) Préparation du bromure de 3-méthyl-1-[2-(4-nitro-phénylamino)-éthyl]-3H-imidazol-1-ium

On a réalisé une suspension de 49,0 g (0.2 mole) de (2-bromo-éthyl)-(4-nitrophényl)-amine et de 19,8 g (0,24 mole) de 1-méthyl-1H-imidazole dans 200 ml de toluène. On a chauffé sous agitation au reflux du toluène pendant 4 heures, essoré bouillant et réempaté deux fois dans l'acétate d'éthyle puis dans l'éthanol absolu.
Après séchage à 40°C sous vide, on a obtenu des cristaux jaune pâle (62,3 g) de bromure de 3-Méthyl-1-[2-(4-nitro-phénylamino)-éthyl]-3H-imidazol-1-ium qui ont fondu à 214°C (Kofler) et dont l'analyse élémentaire calculée pour C₁₂H₁₅N₄O₂Br était :

| % | C | H | N | O | Br |
|---|---|---|---|---|---|
| Calculé | 44,05 | 4,62 | 17,12 | 9,78 | 24,42 |
| Trouvé | 44,14 | 4,57 | 17,03 | 9,78 | 24,37 |

### b) Réduction du bromure de 3-méthyl-1-[2-(4-nitro-phénylamino)-éthyl]-3H-imidazol-1-ium

On a chauffé au reflux de l'alcool un mélange de 50 ml d'éthanol à 96°, 5 ml d'eau, 25 g de zinc en poudre fine et 0,5 g de chlorure d'ammonium. On a ajouté par portions de façon à maintenir le reflux sans chauffage 16,4 g (0,05 mole) de bromure de 3-méthyl-1-[2-(4-nitro-phénylamino)-éthyl]-3H-imidazol-1-ium obtenu à l'étape précédente. La réaction a été exothermique.
A la fin de l'addition on a maintenu le reflux pendant 10 minutes supplémentaires.
On a filtré bouillant en coulant dans 22 ml d'éthanol absolu chlorhydrique (glacé) environ 5N.
Le précipité cristallisé a été essoré, lavé à l'éthanol absolu et séché sous vide à 40°C sur potasse.
On a obtenu, après recristallisation d'un mélange d'eau et d'éthanol au reflux, 10,4 g de cristaux blancs fondant à 195-200°C (Kofler) et dont la structure était conforme en RMN 1H.

### EXEMPLE DE PREPARATION 2 : Synthèse du monochlorure, dichlorhydrate de 1-[3-(2,5-diamino-phénoxy)-propyl]-3-méthyl-3H-imidazol-1-ium

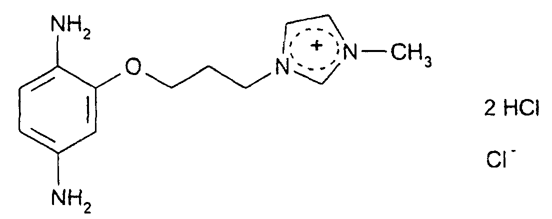

### a) Préparation du N-[2-(3-chloro-propoxy)-4-nitro-phényl]acétamide

On a chauffé sous agitation à 50 °C, un mélange de 98,1 g (0,5 mole) de N-(2-hydroxy-4-nitro-phényl)-acétamide et de 69,2 g (0,5 mole) de carbonate de potassium dans 500 ml de diméthylformamide, puis on a ajouté 113,0 g (1 mole) de 1,3-dichloro-propane et continué à chauffer à 50°C pendant une heure.
On a versé le mélange réactionnel dans 4 litres d'eau glacée, essoré le précipité cristallisé, réempaté dans l'eau puis dans l'alcool isopropylique et séché sous vide à 40°C sur anhydride phosphorique.
On a obtenu 113,5 g de cristaux beiges qui, après purification par recristallisation de l'acétate d'isopropyle au reflux, ont fondu à 121°C.
L'analyse élémentaire était conforme à celle calculée pour C₁₁H₁₃N₂O₄Cl.

### b) Préparation du chlorure de 1-[3-(2-acétylamino-5-nitro-phénoxy)-propyl]-3-méthyl-3H-imidazol-1-ium

On a utilisé le mode opératoire décrit ci-dessus pour l'exemple 1, étape a).
A partir de 27,2 g (0,1 mole) de N-[2-(3-chloro-propoxy)-4-nitro-phényl]-acétamide obtenu à l'étape précédente et de 9,9 g (0,12 mole) de1-méthyl-1H-imidazole dans 120 mi de toluène, on a obtenu des cristaux jaune pâle (21,5 g) de chlorure de 1-[3-acétylamino-5-nitro-phénoxy)-propyl]-3-méthyl-3H-imidazol-1ium qui ont fondu à 227°C (Kofler) et dont l'analyse élémentaire calculée pour C₁₅H₁₉N₄O₄Cl était :

| % | C | H | N | O | Cl |
|---|---|---|---|---|---|
| Calculé | 50,78 | 5,40 | 15,79 | 18,04 | 9,99 |
| Trouvé | 50,69 | 5,36 | 15,74 | 18,23 | 9,79 |

### c) Réduction du chlorure de 1-[3-(2-acétylamino-5-nitro-phénoxy)-propyl]-3-méthyl-3H-imidazol-1-ium

La réduction a été effectuée selon le mode opératoire décrit pour l'exemple 1, étape b).
A partir de 21,3 g (0,06 mole) de chlorure de 1-[3-(2-acétylamino-5-nitrophénoxy)-propyl]-3-méthyl-3H-imidazol-1-ium on a obtenu, après filtration et évaporation à sec sous pression réduite, 19,0g d'une huile brune de chlorure de 1-[3-(2-Acétylamino-5-amino-phénoxy)-propyl]-3-méthyl-3H-imidazol-1-ium.

### d) désacétylation du chlorure de 1-[3-(2-acétylamino-5-amino-phénoxy)-propyl]-3-méthyl-3H-imidazol-1-ium

Le chlorure de 1-[3-(2-acétylamino-5-amino-phénoxy)-propyl]-3-méthyl-3H-imidazol-1-ium, obtenu à l'étape précédente (19,0 g), a été mis en solution, à température ambiante et sous agitation, dans 90 ml d'éthanol absolu chlorhydrique environ 5N.
Au bout d'une demi-heure un précipité cristallisé blanc est apparu.
La suspension a été chauffée une heure au reflux de l'alcool.
On a refroidi, essoré, lavé à l'éthanol absolu et séché à 50°C sous vide et sur potasse.
On a obtenu 14,9 g de cristaux blanc cassé qui ont fondu à 216-220°C (Kofler) et dont l'analyse élémentaire calculée pour C₁₃H₂₁N₄OCl₃ était :

| % | C | H | N | O | Cl |
|---|---|---|---|---|---|
| Calculé | 43,90 | 5,95 | 15,75 | 4,50 | 29,90 |
| Trouvé | 43,83 | 6,01 | 15,62 | 5,09 | 29,80 |

### EXEMPLE DE PREPARATION 3 : Synthèse du monochlorure, dichlorhydrate de 3-[3-(4-amino-phénylamino)-propyl]-1-méthyl-3H-imidazol-1-ium

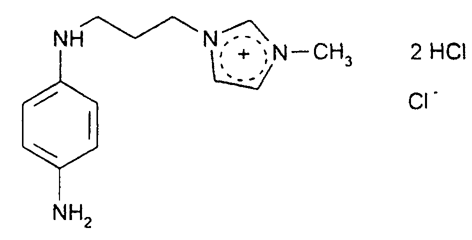

### a) Préparation de la (3-imidazol-1-yl-propyl)-(4-nitro-phényl)-amine

Sous agitation, on a chauffé pendant une demi-heure un mélange de 28,2 g (0,2 mole) de 1-fluoro-4-nitro-benzène, de 31,3 g (0,25 mole) de 3-imidazol-1-yl-propylamine et de 34,8 ml de triéthylamine dans 30 ml de 1,2-diméthoxy-éthane. On a versé dans 1,5 litres d'eau glacée, essoré le précipité cristallisé, réempaté dans l'eau puis dans l'alcool isopropylique et séché sous vide à 40°C sur anhydride phosphorique. On a obtenu des cristaux jaunes (36,6 g) qui, après purification par recristallisation de l'éthanol à 96° au reflux, ont fondu à 124°C et dont l'analyse élémentaire calculée pour C₁₂H₁₄N₄O₂ était :

| % | C | H | N | O |
|---|---|---|---|---|
| Calculé | 58,53 | 5,73 | 22,75 | 12,99 |
| Trouvé | 58,17 | 5,75 | 22,67 | 13,45 |

### b) Quaternisation de la (3-imidazol-1-yl-propyl)-(4-nitro-phényl)-amine

On a fait la suspension de 30,4 g (0,123 mole) de (3-imidazol-1-yl-propyl)-(4-nitro-phényl)-amine obtenu à l'étape précédente et de 12,9 ml de diméthylsulfate dans 600 ml d'acétate d'éthyle, que l'on a laissé pendant 2 heures à température ambiante sous agitation.

Le précipité cristallisé a été essoré, lavé plusieurs fois dans l'acétate d'éthyle, réempaté dans le minimum d'éthanol absolu et séché sous vide à 50°C.
on a obtenu 37,6 g de cristaux jaunes qui ont fondu à 74°C (Kofler) et dont l'analyse élémentaire calculée pour C₁₄H₂₀N₄O₆S était :

| % | C | H | N | O | S |
|---|---|---|---|---|---|
| Calculé | 45,15 | 5,41 | 15,04 | 25,78 | 8,61 |
| Trouvé | 44,85 | 5,50 | 14,91 | 25,97 | 8,49 |

### c) Réduction du méthylsulfate de 1-méthyl-3-[3-(4-nitro-phénylamino)-propyl]-3H-imidazol-1-ium

La réduction a été effectuée selon le mode opératoire décrit pour l'exemple 1, étape b).
A partir de 33,5 g (0,09 mole) de méthylsulfate de 1-méthyl-3-[3-(4-nitro-phénylamino)-propyl]-3H-imidazol-1-ium obtenu ci-dessus à l'étape précédente, on a obtenu, après chauffage dans l'éthanol absolu chlorhydrique environ 5N pour compléter l'échange d'anions, 18,7 g de cristaux blancs qui ont fondu avec décomposition à 184-190°C (Kofler) et dont l'analyse élémentaire calculée pour C₁₃H₂₁N₄Cl₃ + 1/3H₂O était :

| % | C | H | N | O | Cl |
|---|---|---|---|---|---|
| Calculé | 45,17 | 6,32 | 16,21 | 1,54 | 30,77 |
| Trouvé | 44,98 | 6,22 | 16,05 | 1,57 | 30,78 |

### EXEMPLE DE PREPARATION 4 : Synthèse du monochlorure, dichlorhydrate de 3-[3-(4-amino-3-méthyl-phénylamino)-propyl]-1-méthyl-3H-imidazol-1-ium

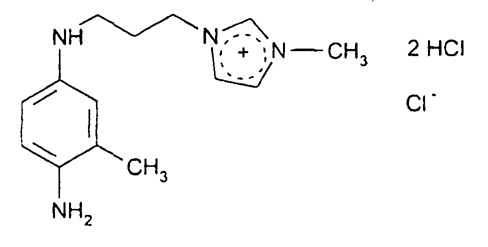

### a) Préparation de la (3-imidazol-1-yl-propyl)-(3-méthyl-4-nitro-phényl)-amine

Sous agitation on a chauffé pendant 3 heures au bain-marie bouillant un mélange de 31,2 g (0,2 mole) de 4-fluoro-2-méthyl-1-nitro-benzène, de 37,5 g (0,3 mole) de 3-imidazol-1-yl-propylamine et de 34,8 ml (0,25 mole) de triéthylamine dans 30 ml de 1,2-diméthoxy-éthane.
On a versé dans 0,5 l d'eau glacée, essoré le précipité cristallisé, réempaté dans l'eau puis dans l'alcool isopropylique et séché sous vide à 40°C sur anhydride phosphorique.
Après purification par recristallisation de l'éthanol à 96° au reflux, on a obtenu 17,0 g de cristaux jaune orangé qui ont fondu à 133°C (Kofler) et dont l'analyse élémentaire calculée pour C₁₃H₁₆N₄O₂ était :

| % | C | H | N | O |
|---|---|---|---|---|
| Calculé | 59,99 | 6,20 | 21,52 | 12,29 |
| Trouvé | 59,55 | 6,22 | 21,43 | 12,88 |

### b) Préparation du méthylsulfate de 1-méthyl-3-[3-(3-méthyl-4-nitro-phénylamino)-propyl]-3H-imidazol-1-ium

La quaternisation de 16,5 g (0,063 mole ) de (3-imidazol-1-yl-propyl)-(3-méthyl-4-nitro-phenyl)-amine obtenu ci-dessus à l'étape précédente dissous dans 165 ml d'acétate d'éthyle a été faite en ajoutant 6,7 ml (0,07 mole) de diméthylsulfate sous agitation, pendant une heure, à température ambiante.
On a obtenu 20,8 g d'huile jaune de méthylsulfate de 1-méthyl-3-[3-(3-méthyl-4-nitro-phénylamino)-propyl]-3H-imidazol-1-ium.

### c) réduction du méthylsulfate de 1-méthyl-3-[3-(3-méthyl-4-nitro-phénylamino)-propyl]-3H-imidazol-1-ium

La réduction a été effectuée selon le mode opératoire décrit ci-dessus pour l'exemple 1, étape b).
A partir de 20,0 g (0,051 mole) de méthylsulfate de 1-méthyl-3-[3-(3-méthyl-4-nitro-phénylamino)-propyl]-3H-imidazol-1-ium obtenu ci-dessus à l'étape précédente, on a obtenu, après chauffage dans l'éthanol absolu chlorhydrique environ 5N pour compléter l'échange d'anions, 12,5 g de cristaux blancs qui ont fondu à 210-220°C (Kofler) et dont l'analyse élémentaire calculée pour C₁₄H₂₃N₄Cl₃ + ½ H₂O était :

| % | C | H | N | O | Cl |
|---|---|---|---|---|---|
| Calculé | 46,36 | 6,67 | 15,45 | 2,21 | 29,32 |
| Trouvé | 46,21 | 6,40 | 15,33 | 2,37 | 29,69 |

### EXEMPLE DE PREPARATION 5 : Synthèse du monochlorure, dichlorhydrate de 3-[3-(4-amino-2-méthyl-phénylamino)-propyl]-1-méthyl-3H-imidazol-1-ium

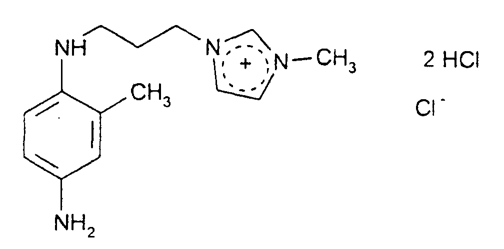

### a) Préparation de la (3-imidazol-1-yl-propyl)-(2-méthyl-4-nitro-phényl)-amine

On utilise le mode opératoire décrit pour l'exemple 4, étape a).
A partir de 31,2 g (0,2 mole) de 1-fluoro-2-méthyl-4-nitro-benzène et de 37,5 g (0,3 mole) de 3-imidazol-1-yl-propylamine, et après purification par recristallisation de l'éthanol à 96° au reflux, on a obtenu 23,0 g de cristaux jaune orangé qui ont fondu à 163°C (Kofler) et dont l'analyse élémentaire calculée pour C₁₃H₁₆N₄O₂ + ¼ H₂O était :

| % | C | H | N | O |
|---|---|---|---|---|
| Calculé | 58,97 | 6,28 | 21,16 | 13,59 |
| Trouvé | 59,10 | 6,22 | 21,09 | 12,85 |

### b) Préparation du méthylsulfate de 1-méthyl-3-[3-(2-méthyl-4-nitro-phénylamino)-propyl]-3H-imidazol-1-ium

On utilise le mode opératoire décrit pour l'exemple 4, étape b).
A partir de 22,5 g (0,086 mole ) de (3-imidazol-1-yl-propyl)-(2-méthyl-4-nitrophényl)-amine obtenu à l'étape précédente et de 9,0 ml (0,095 mole) de sulfate de méthyle, on a obtenu 19,5 g de cristaux jaunes de méthylsulfate de 1-méthyl-3-[3-(2-méthyl-4-nitro-phénylamino)-propyl]-3H-imidazol-1-ium qui ont fondu à 70°C (Kofier) et dont l'analyse élémentaire calculée pour C₁₄H₁₉N₄O₂ était :

| % | C | H | N | O | S |
|---|---|---|---|---|---|
| Calculé | 46,62 | 5,74 | 14,50 | 24,84 | 8,30 |
| Trouvé | 46,66 | 5,80 | 14,50 | 24,90 | 8,27 |

### c) Réduction du méthylsulfate de 1-méthyl-3-[3-(2-méthyl-4-nitro-phénylamino)-propyl]-3H-imidazol-1-ium

La réduction est effectuée selon le mode opératoire décrit pour l'exemple 1, étape b).
A partir de 19,0 g (0,05 mole) de méthylsulfate de 1-méthyl-3-[3-(2-méthyl-4-nitro-phénylamino)-propyl]-3H-imidazol-1-ium, on a obtenu, après chauffage dans l'éthanol absolu chlorhydrique environ 5N pour compléter l'échange d'anions, 14,6 g de cristaux blancs qui ont fondu à 255-260°C (Kofler) et dont l'analyse élémentaire calculée pour C₁₄H₂₃N₄Cl₃ + ½ H₂O était :

| % | C | H | N | O | Cl |
|---|---|---|---|---|---|
| Calculé | 46,36 | 6,67 | 15,45 | 2,21 | 29,32 |
| Trouvé | 45,84 | 6,63 | 15,35 | 2,09 | 29,67 |

### EXEMPLE DE PREPARATION 6 : Synthèse du monochlorure, dichlorhydrate, monohydrate de 3-[3-(4-amino-2-fluoro-phénylamino)-propyl]-1-méthyl-3H-imidazol-1-ium

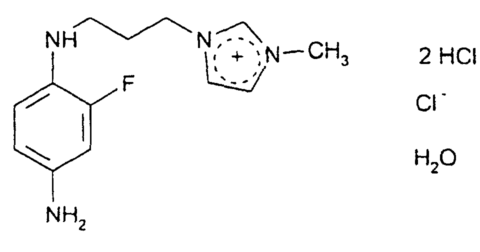

### a) Préparation de la (2-fluoro-4-nitro-phényl)-(3-imidazol-1-yl-propyl)-amine

On utilise le mode opératoire décrit ci-dessus pour l'exemple 4, étape a).
A partir de 31,8 g (0,2 mole) de 1,2-difluoro-4-nitro-benzène et de 37,5 g (0,3 mole) de 3-imidazol-1-yl-propylamine, et après purification par recristallisation de l'éthanol à 96° au reflux, on a obtenu 36,0g de cristaux jaune orangé qui ont fondu à 144°C (Kofler) et dont l'analyse élémentaire calculée pour C₁₂H₁₃N₄O₂F était :

| % | C | H | N | O | F |
|---|---|---|---|---|---|
| Calculé | 54,54 | 4,96 | 21,20 | 12,11 | 7,19 |
| Trouvé | 54,25 | 4,99 | 21,14 | - | 6,97 |

### b) Préparation du méthylsulfate de 3-[3-(2-fluoro-4-nitro-phénylamino)-propyl]-1-méthyl-3H-imidazol-1-ium

On utilise le mode opératoire décrit pour l'exemple 4, étape b).
A partir de 36,0 g (0,136 mole) de (2-fluoro-4-nitro-phényl)-(3-imidazol-1-yl-propyl)-amine obtenu à l'étape précédente et de 14,3 ml (0,15 mole) de sulfate de méthyle, on a obtenu 46,0 g de cristaux jaunes de méthylsulfate de 3-[3-(2-fluoro-4-nitro-phénylamino)-propyl]-1-méthyl-3H-imidazol-1-ium qui ont fondu avec décomposition à 110°C (Kofler) et dont l'analyse élémentaire calculée pour C₁₄H₁₉N₄O₆SF était :

| % | C | H | N | O | S | F |
|---|---|---|---|---|---|---|
| Calculé | 43,07 | 4,91 | 14,35 | 24,59 | 4,87 | 8,21 |
| Trouvé | 43,00 | 5,00 | 14,37 | - | 4,87 | 8,12 |

### c) Réduction du méthylsulfate de 3-[3-(2-fluoro-4-nitro-phénylamino)-propyl]-1-méthyl-3H-imidazol-1-ium

La réduction a été effectuée selon le mode opératoire décrit ci-dessus pour l'exemple 1, étape b).
A partir de 41,0 g (0,105 mole) de méthylsulfate de 3-[3-(2-fluoro-4-nitrophénylamino)-propyl]-1-méthyl-3H-imidazol-1-ium on a obtenu, après chauffage dans l'éthanol absolu chlorhydrique environ 5N pour compléter l'échange d'anions, 19,0 g de cristaux blancs qui ont fondu avec décomposition à 165-170°C (Kofler) et dont l'analyse élémentaire calculée pour C₁₃H₂₀N₄Cl₃F + H₂O était :

| % | C | H | N | O | Cl | F |
|---|---|---|---|---|---|---|
| Calculé | 41,56 | 5,90 | 14,91 | 4,26 | 28,31 | 5,06 |
| Trouvé | 41,59 | 5,41 | 14,88 | - | 29,13 | 5,32 |

### EXEMPLE DE PREPARATION 7 : Synthèse du monochlorure, chlorhydrate de 3-[3-(4-amino-2-cyano-phénylamino)-propyl]-1-méthyl-3H-imidazol-1-ium

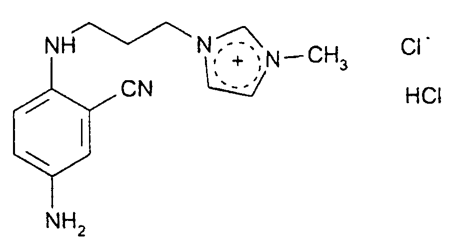

### a) Préparation du 2-(3-imidazol-1-yl-propylamino)-5-nitro-benzonitrile

On a utilisé le mode opératoire décrit pour l'exemple 4, étape a), mais en utilisant de la N-méthylpyrrolidone à la place du 1,2-diméthoxy-ethane.
A partir de 36,5 g (0,2 mole) de 2-chloro-5-nitro-benzonitrile et de 31,3 g (0,25 mole) de 3-imidazol-1-yl-propylamine, et après purification par recristallisation de l'éthanol à 96° au reflux, on a obtenu 28,2 g de cristaux jaunes qui ont fondu à 177°C (Kofler) et dont l'analyse élémentaire calculée pour C₁₃H₁₃N₅O₂ était :

| % | C | H | N | O |
|---|---|---|---|---|
| Calculé | 57,56 | 4,83 | 25,82 | 11,80 |
| Trouvé | 57,69 | 4,86 | 25,65 | 11,94 |

### b) Préparation du méthylsulfate de 3-[3-(2-cyano-4-nitro-phénylamino)-propyl]-1-méthyl-3H-imidazol-1-ium

On a utilisé le mode opératoire décrit pour l'exemple 4, étape b).
A partir de 27,7 g (0,102 mole) de 2-(3-imidazol-1-yl-propylamino)-5-nitro-benzonitrile obtenu à l'étape précédente et de 10,8 ml (0,114 mole) de sulfate de méthyle, et après purification par recristallisation de l'éthanol absolu, on a obtenu 30,0 g de cristaux jaunes de méthylsulfate de 3-[3-(2-cyano-4-nitro-phénylamino)-propyl]-1-méthyl-3H-imidazol-1-ium qui ont fondu à 110-115°C (Kofler) et dont l'analyse élémentaire calculée pour C₁₅H₁₉N₅O₆S était :

| % | C | H | N | O | S |
|---|---|---|---|---|---|
| Calculé | 45,34 | 4,82 | 17,62 | 24,16 | 8,07 |
| Trouvé | 45,31 | 4,82 | 17,73 | 24,21 | 8,15 |

### c) Réduction du méthylsulfate de 3-[3-(2-cyano-4-nitro-phénylamino)-propyl]-1-méthyl-3H-imidazol-1-ium

La réduction est effectuée selon le mode opératoire décrit ci-dessus pour l'exemple 1, étape b).
A partir de 25,0 g (0,063 mole) de méthylsulfate de 3-[3-(2-cyano-4-nitro-phénylamino)-propyl]-1-méhyl-3H-imidazol-1-ium on a obtenu, après chauffage dans l'éthanol absolu chlorhydrique environ 5N pour compléter l'échange d'anions, 16,2 g de cristaux blancs qui ont fondu à 220°C (Kofler) et dont l'analyse RMN 1 H était conforme au produit attendu (NH non salifié).

### EXEMPLE DE PREPARATION 8 : Synthèse du monochlorure, dichlorhydrate de 1-[2-(4-amino-2-méthoxy-phénylamino)-éthyl]-3-méthyl-3H-imidazol-1-ium

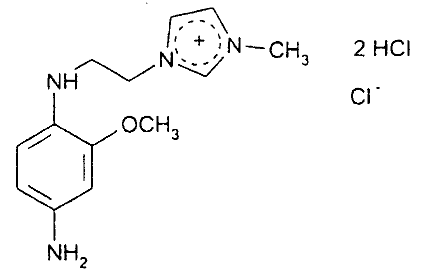

### a) Préparation du bromure de 1-[2-(2-méthoxy-4-nitro-phénylamino)-éthyl]-3-méthyl-3H-imidazol-1-ium

On a chauffé 7 heures au reflux un mélange de 46,8 g (0,17 mole) de (2-bromoéthyl)-(2-méthoxy-4-nitro-phényl)-amine et de 20,5 g (0,25 mole) de 1-méthyl-1H-imidazole dans 170 ml de toluène.
On a essoré le précipité cristallisé, réempaté dans l'éthanol absolu et séché sous vide à 50°C.

On a obtenu 50,2 g de cristaux jaunes qui ont fondu à 184°C (Kofler) et dont l'analyse élémentaire calculée pour C₁₃H₁₇N₄O₃Br était :

| % | C | H | N | O | Br |
|---|---|---|---|---|---|
| Calculé | 43,71 | 4,80 | 15,68 | 13,44 | 22,37 |
| Trouvé | 43,59 | 4,85 | 15,66 | 14,25 | 22,03 |

### b) Réduction du bromure de 1-[2-(2-méthoxy-4-nitro-phénylamino)-éthyl]-3-méthyl-3H-imidazol-1-ium

La réduction est effectuée selon le mode opératoire décrit ci-dessus pour l'exemple 1, étape b).
A partir de 39,5 g (0,11 mole) de bromure de 1-[2-(2-méthoxy-4-nitro-phénylamino)-éthyl]-3-méthyl-3H-imidazol-1-ium on a obtenu, après chauffage dans l'éthanol absolu chlorhydrique environ 5N pour compléter l'échange d'anions, 12,5 g de cristaux légèrement gris qui ont fondu avec décomposition à 210-218°C (Kofler) et dont l'analyse élémentaire calculée pour C₁₃H₂₁N₄OCl₃ + ½ H₂O était :

| % | C | H | N | O | Cl |
|---|---|---|---|---|---|
| Calculé | 42,81 | 6,08 | 15,36 | 6,58 | 29,16 |
| Trouvé | 42,42 | 5,99 | 14,88 | 6,14 | 29,55 |

### EXEMPLE DE PREPARATION 9 : Synthèse du monochlorure, chlorhydrate de 1-(5-amino-2-hydroxy-benzyl)-3-méthyl-3H-imidazol-1-ium

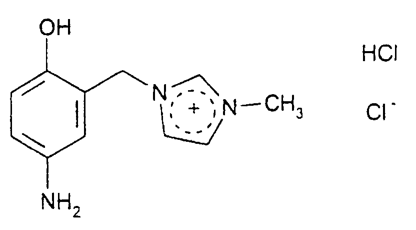

### a) Préparation du chlorure de 1-(2-hydroxy-5-nitro-benzyl)-3-méthyl-3H-imidazol-1-ium

On utilise le mode opératoire décrit pour l'exemple 8, étape a).
A partir de 56,3 g (0,3 mole) de 2-chlorométhyl-4-nitro-phénol et de 29,6 g (0,36 mole) de 1-méthyl-1H-imidazole on a obtenu 65,1 g de cristaux jaunes qui ont fondu avec décomposition à 250-260°C (Kofler) et dont l'analyse élémentaire calculée pour C₁₁H₁₂N₃O₃Cl était :

| % | C | H | N | O | Cl |
|---|---|---|---|---|---|
| Calculé | 48,99 | 4,49 | 15,58 | 17,80 | 13,15 |
| Trouvé | 48,74 | 4,58 | 15,72 | 17,62 | 13,27 |

### b) Réduction du chlorure de 1-(2-hydroxy-5-nitro-benzyl)-3-méthyl-3H-imidazol-1-ium

Dans un hydrogénateur on a placé 27,5 g (0,102 mole) de chlorure de 1-(2-hydroxy-5-nitro-benzyl)-3-methyl-3H-imidazol-1-ium obtenu à l'étape précédente, 10 g de palladium à 5% sur charbon (contenant 50% d'eau), et 400 ml d'eau.
La réduction s'est faite en une heure sous une pression d'hydrogène d'environ 4 bars et à une température qui a été portée progressivement à 35°C.
Après filtration du catalyseur sous azote on a coulé sur de l'acide chlorhydrique aqueux.
On a évaporé le filtrat à sec sous pression réduite, repris dans l'éthanol absolu et essoré.
Après séchage à 40°C sous vide et sur potasse on a obtenu 23,5 g de cristaux blancs qui ont fondu à 170-175°C (Kofler) et dont l'analyse élémentaire était conforme à celle calculée pour C₁₁H₁₅N₃OCl₂.
La structure était conforme en RMN 1H.

### EXEMPLE DE PREPARATION 10 : Synthèse du chlorure de 3-[2-(2,5-diamino-phényl)-éthyl]-1-méthyl-3H-imidazol-1-ium, dichlorhydrate

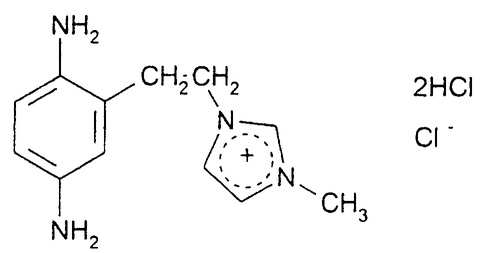

### a) Préparation du N-[4-acétylamino-2-(2-chloro-éthyl)-phényl]-acétamide

On a mis en solution 135,0 g (0,6 mole) de dichlorhydrate de 2-(2,5-diamino-phényl)-éthanol dans 700 ml d'eau à température ambiante et ajouté une solution de 83,2 g (0,66 mole) de sulfite de sodium dans 166 ml d'eau.
On a coulé rapidement 140,8 ml (1,5 moles) d'anhydride acétique (réaction exothermique) et agité pendant deux heures la suspension.
On a essoré, lavé à l'eau et séché sous vide à 45°C sur anhydride phosphorique.
On a obtenu 115,1 g de cristaux blancs de N-[4-acétylamino-2-(2-hydroxyéthyl)-phényl]-acétamide qui ont fondu à 202°C.
On a dissous, à température ambiante, 64,1 g (0,272 mole) de ce composé dans 500 ml de diméthylformamide et 53,0 ml (0,38 mole) de triéthylamine.
On a refroidi à environ 0°C et coulé goutte à goutte, sous agitation et en maintenant la température entre 0 et 5°C, 25,3 ml (0,326 mole) de chlorure de mésyle.
Le chlorhydrate de triéthylamine formé a été filtré et on a ajouté au filtrat 90,0 g (2,12 mole) de chlorure de lithium. Sous agitation, on a chauffé pendant 15 minutes à une température de 110-115°C.
On a versé dans 1 kg d'eau glacée, essoré le précipité cristallisé, lavé à l'eau et recristallisé de l'isopropanol au reflux.

On a obtenu 53,4 g de cristaux blancs de N-[4-acétylamino-2-(2-chloro-éthyl)-phényl]-acétamide qui ont fondu à 214-216°C et dont l'analyse élémentaire calculée pour C₁₂H₁₅N₂O₂Cl était :

| % | C | H | N | O | Cl |
|---|---|---|---|---|---|
| Calculé | 56,59 | 5,94 | 11,00 | 12,56 | 13,92 |
| Trouvé | 56,31 | 6,05 | 11,10 | 12,95 | 13,92 |

### b) Quaternisation et désacétylation

On a chauffé au reflux pendant 18 heures un mélange de 25,5 g (0,1 mole) de N-[4-acétylamino-2-(2-chloro-éthyl)-phényl]-acétamide obtenu ci-dessus à l'étape précédente et de 17,5 ml (0,22 mole) de 1-méthyl-1H-imidazole dans 150 ml de toluène et 210 ml d'isobutanol.
On a évaporé à sec sous pression réduite.
La gomme de 3-[2-(2,5-bis-acétylamino-phényl)-éthyl]-1-méthyl-3H-imidazol-1-ium obtenue a ensuite été chauffée pendant 6 heures au reflux dans 100 ml d'acide chlorhydrique aqueux à 36%.
On a évaporé à sec sous pression réduite, repris dans l'isopropanol et essoré le précipité cristallisé.
Après séchage à 40°C sous vide et sur anhydride phosphorique, on a obtenu 24,7g de cristaux crème de chlorure de 3-[2-(2,5-diamino-phényl)-éthyl]-1-méthyl-3H-imidazol-1-ium, dichlorhydrate qui ont fondu avec décomposition à plus de 260°C (Kofler) et dont l'analyse élémentaire calculée pour C₁₂H₁₉N₄Cl₃ + ½ H₂O était :

| % | C | H | N | O | Cl |
|---|---|---|---|---|---|
| Calculé | 43,07 | 6,02 | 16,74 | 2,39 | 31,78 |
| Trouvé | 43,29 | 6,25 | 16,62 | 2,21 | 32,06 |

### EXEMPLE DE PREPARATION 11 : Synthèse du chlorure de 1-{2-[(4-amino-phényl)-éthylamino]-éthyl}-3-méthyl-3H-imidazol-1-ium, dichlorhydrate

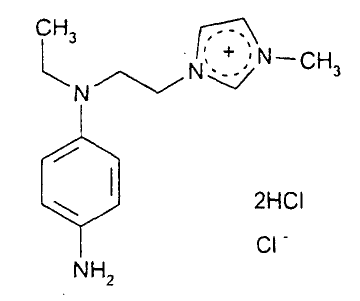

### a) Préparation du N-{4-[(2-chloro-éthyl)-éthylamino]-phényl}-acétamide.

On a dissous à température ambiante 66,7 g (0,3 mole) de N-{4-[éthyl-(2-hydroxyéthyl)-amino]-phényl}-acétamide dans 500 ml de diméthylformamide et 58,5 ml (0,42 mole) de triéthylamine.
On a refroidi à environ 0°C et coulé goutte à goutte, sous agitation et en maintenant la température entre 0 et 5°C, 28,0 ml (0,36 mole) de chlorure de mésyle.
Le chlorhydrate de triéthylamine formé a été filtré et on a ajouté au filtrat 38,2 g (0,9 mole) de chlorure de lithium.
Sous agitation, on a chauffé pendant 15 minutes à 100-108°C.
On a versé dans 1 kg d'eau glacée, essoré le précipité cristallisé, lavé à l'eau et recristallisé de l'isopropanol au reflux.
On a obtenu 62,7 g de cristaux blancs de N-{4-[(2-chloro-éthyl)-éthylamino]-phényl}-acétamide qui ont fondu à 102°C et dont l'analyse élémentaire calculée pour C₁₂H₁₇N₂OCl était :

| % | C | H | N | O | Cl |
|---|---|---|---|---|---|
| Calculé | 59,87 | 7,12 | 11,64 | 6,65 | 14,73 |
| Trouvé | 59,42 | 7,10 | 11,33 | 7,54 | 14,42 |

### b) Quaternisation du N-{4-[(2-chloro-éthyl)-éthylamino]-phényl}-acétamide

On a chauffé au reflux pendant 4 heures un mélange de 24,1 g (0,1 mole) de N-{4-[(2-chloro-éthyl)-éthylamino]-phényl}-acétamide obtenu ci-dessus à l'étape précédente et de 17,5 ml (0,22 mole) de 1-méthyl-1H-imidazole dans 70 ml d'isobutanol.
On a refroidi vers 0°C et ajouté 140 ml de toluène.
Le précipité cristallisé a été essoré, lavé dans le toluène puis l'éther de pétrole, et séché sous vide à 45°C et sur anhydride phosphorique.
On a obtenu 31,5 g de cristaux blancs de chlorure de 3-{2-[(4-acétylamino-phényl)-éthylamino]-éthyl}-1-méthyl-3H-imidazol-1-ium qui ont fondu à 206°C et dont l'analyse élémentaire calculée pour C₁₆H₂₃N₄OCl + ¼ H₂O était :

| % | C | H | N | O | Cl |
|---|---|---|---|---|---|
| Calculé | 58,71 | 7,24 | 17,12 | 6,11 | 10,83 |
| Trouvé | 58,77 | 7,18 | 17,25 | 6,05 | 10,68 |

### c) Désacétylation du 3-{2-[(4-acétylamino-phényl)-éthylamino]-éthyl}-1-méthyl-3H-imidazol-1-ium

On a chauffé au reflux pendant 1 heure 29 g (0,09 mole) de chlorure de 3-{2-[(4-acétylamino-phényl)-éthylamino]-éthyl}-1-méthyl-3H-imidazol-1-ium obtenu ci-dessus à l'étape précédente dans 30 ml d'acide chlorhydrique 36%. On a évaporé à sec sous pression réduite, repris dans l'éthanol absolu, précipité par dilution à l'éther éthylique, essoré et séché le précipité cristallisé. On a obtenu 13,4 g de cristaux blancs de chlorure de 1-{2-[(4-amino-phényl)-éthylamino]-éthyl}-3-méthyl-3H-imidazol-1-ium, dichlorhydrate qui ont fondu avec décomposition à 212-214°C (Kofler) et dont la RMN 1H était conforme à celle du produit attendu.

### EXEMPLE DE PREPARATION 12 : Synthèse du dichlorure de N,N-bis-[2-(3-methyl-3H-imidazol-1-ium)-éthyl]-4-amino-aniline, monochlorhydrate, monohydrate

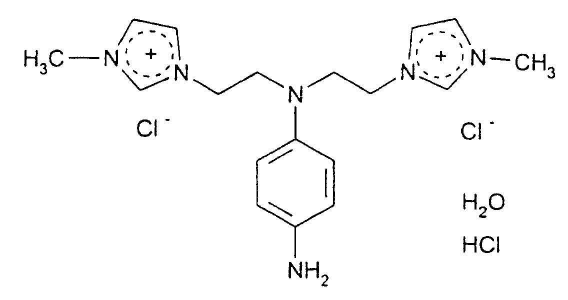

### a) Préparation du dichlorure de N,N-bis-[2-(3-méthyl-3H-imidazol-1-ium)-éthyl]-4-nitro-aniline, dihydrate

On a chauffé au reflux pendant 6 heures un mélange de 31,5 g (0,12 mole) de bis-(2-chloro-éthyl)-(4-nitro-phényl)-amine et de 59,1 g (0,72 mole) de 1-méthyl-1H-imidazole dans 60 ml de toluène.
Le précipité cristallisé formé a été essoré à chaud, lavé dans le toluène et recristallisé d'un mélange d'eau et d'éthanol au reflux.
On a obtenu 45,0 g de cristaux jaunes de dichlorure de N,N-bis-[2-(3-méthyl-3H-imidazol-1-ium)-éthyl]-4-nitro-aniline, dihydrate qui ont fondu avec décomposition à plus de 260°C et dont l'analyse élémentaire calculée pour C₁₈H₂₄N₆O₂Cl₂ +2 H₂O était :

| % | C | H | N | O | Cl |
|---|---|---|---|---|---|
| Calculé | 46,66 | 6,09 | 18,14 | 13,81 | 15,30 |
| Trouvé | 46,72 | 6,20 | 18,12 | 13,85 | 15,25 |

### b) Réduction du dichlorure de N,N-bis-[2-(3-méthyl-3H-imidazol-1-ium)-éthyl]-4-nitro-aniline, dihydrate

Dans un hydrogénateur, on a placé 45,0 g (0,105 mole) de dichlorure de N,N-bis-[2-(3-méthyl-3H-imidazol-1-ium)-éthyl]-4-nitro-aniline, dihydrate obtenu ci-dessus à l'étape précédente, 16 g de palladium à 5% sur charbon (contenant 50% d'eau), 300 ml d'éthanol et 300 ml d'eau.
La réduction s'est faite en une heure sous une pression d'hydrogène d'environ 8 bars et à une température qui a été portée progressivement à 80°C.
Après filtration du catalyseur sous azote on a coulé sur acide chlorhydrique 36%.
On a évaporé à sec sous pression réduite, repris dans l'éthanol absolu et essoré.
Après recristallisation de l'éthanol à 96° au reflux, on a obtenu 28,2 g de cristaux blancs de dichlorure de N,N-bis-[2-(3-méthyl-3H-imidazol-1-ium)-éthyl]-4-amino-aniline, monochlorhydrate, monohydrate qui ont fondu avec décomposition à plus de 260°C (Kofler), et dont l'analyse élémentaire calculée pour C₁₈H₂₇N₆Cl₃ + H₂O était. :

| % | C | H | N | O | Cl |
|---|---|---|---|---|---|
| Calculé | 47,85 | 6,47 | 18,60 | 3,54 | 2.54 |
| Trouvé | 46,93 | 6,55 | 18,03 | | 23,72 |

### EXEMPLE DE PREPARATION 13 : Synthèse du chlorure de 3-[2-(4-amino-phénylamino)-butyl]-1-méthyl-3H-imidazol-1-ium, dichlorhydrate

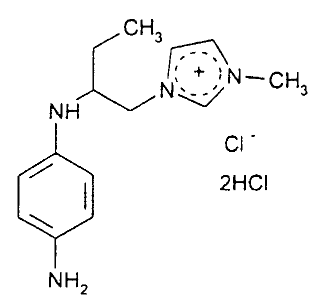

### a) Préparation du 2-(4-nitro-phénylamino)-butan-1-ol

On a chauffé pendant 2 heures au reflux un mélange de 223,0 g (1,58 moles) de 1-fluoro-4-nitro-benzène, de 168,5 g (1,89 moles) de 2-amino-butan-1-ol et de 146,8 g (1,06 moles) de carbonate de potassium dans 630 ml d'eau.
On a refroidi à température ambiante, éliminé la phase aqueuse et repris l'huile orangée dans l'acétate d'éthyle.
Après lavage à l'eau de la phase acétate d'éthyle, séchage sur sulfate de sodium anhydre, filtration, évaporation à sec sous pression réduite et recristallisation de l'éthanol à 96° au reflux, on a obtenu 84,4 g de cristaux orangés de 2-(4-nitro-phénylamino)-butan-1-ol qui ont fondu à 90°C (Kofler) et dont l'analyse élémentaire calculée pour C₁₀H₁₄N₂O₃ était :

| % | C | H | N | O |
|---|---|---|---|---|
| Calculé | 57,13 | 6,71 | 13,32 | 22,83 |
| Trouvé | 57,17 | 6,73 | 13,36 | 22,75 |

### b) Préparation de la (1-chlorométhyl-propyl)-(4-nitro-phényl)-amine

On a utilisé le mode opératoire décrit ci-dessus à l'exemple 11, étape a ).
A partir de 63,1 g (0,3 mole) de 2-(4-nitro-phénylamino)-butan-1-ol obtenu ci-dessus à l'étape précédente on a obtenu, après recristallisation de l'éthanol à 90° au reflux, 47,8 g de cristaux jaunes de (1-chlorométhyl-propyl)-(4-nitro-phényl)-amine qui ont fondu à 50-52°C et dont l'analyse élémentaire calculée pour C₁₀H₁₃N₂O₂Cl était :

| % | C | H | N | O | Cl |
|---|---|---|---|---|---|
| Calculé | 52,52 | 5,73 | 12,25 | 13,99 | 15.50 |
| Trouvé | 52,46 | 5,89 | 12,14 | 13,91 | 15,55 |

### c) Préparation du chlorure de 1-méthyl-3-[2-(4-nitro-phénylamino)-butyl]-3H-imidazol-1-ium

On a chauffé au reflux pendant 9 heures un mélange de 22,9 g (0,1 mole) de (1-chlorométhyl-propyl)-(4-nitro-phényl)-amine obtenue ci-dessus à l'étape précédente et de 17,5 ml (0,22 mole) de 1-méthyl-1H-imidazole dans 70 ml de toluène.
Le précipité cristallisé a été essoré, lavé dans le toluène puis l'éther de pétrole et recristallisé de l'isopropanol au reflux.
On a obtenu 16,0 g de cristaux jaunes de chlorure de 1-méthyl-3-[2-(4-nitro-phénylamino)-butyl]-3H-imidazol-1-ium qui ont fondu à 191°C et dont l'analyse élémentaire calculée pour C₁₄H₁₉N₄O₂Cl + ½ H₂O était :

| % | C | H | N | O | Cl |
|---|---|---|---|---|---|
| Calculé | 52,58 | 6,30 | 17,52 | 12,51 | 11,09 |
| Trouvé | 52,03 | 6,23 | 17,01 | 12,76 | 10,94 |

### d) Réduction du chlorure de 1-méthyl-3-[2-(4-nitro-phénylamino)-butyl]-3H-imidazol-1-ium

On a utilisé le mode opératoire décrit ci-dessus à l'exemple 12, étape b).
On a obtenu, après recristallisation d'un mélange d'éthanol 96° et d'acide chlorhydrique 36% au reflux, 18,6 g de cristaux blancs de chlorure de 3-[2-(4-amino-phénylamino)-butyl]-1-méthyl-3H-imidazol-1-ium, dichlorhydrate qui ont fondu à 214-216°C et dont l'analyse élémentaire calculée pour C₁₄H₂₃N₄Cl₃ était :

| % | C | H | N | Cl |
|---|---|---|---|---|
| Calculé | 47,54 | 6,55 | 15,84 | 30,07 |
| Trouvé | 47,02 | 6,69 | 15,71 | 29,50 |

### EXEMPLE DE PREPARATION 14 : Synthèse du chlorure de 1-{[5-amino-2-(2-hydroxy-éthylamino)-phénylcarbamoyl]-méthyl}-3-méthyl-3H-imidazol-1-ium, dichlorhydrate

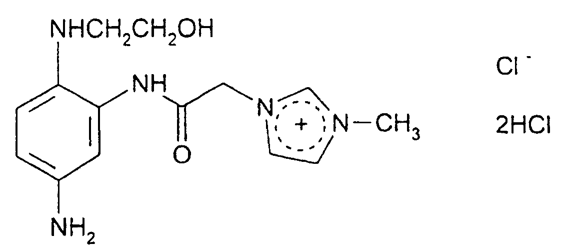

### a) Préparation du 2-chloro-N-[2-(2-hydroxy-éthylamino)-5-nitro-phéenyl]-acétamide

On a refroidi à 5°C un mélange de 82,5 g (0,418 mole) de 2-(2-amino-4-nitro-phénylamino)-éthanol et de 34,6 g (0,25 mole) de carbonate de potassium dans 400 ml de diméthylformamide.

On a ajouté goutte à goutte, en maintenant la température entre 5 et 12°C, 34,7 ml de chlorure de chloracétyle.
On a agité pendant une heure supplémentaire.
On a versé sur un mélange de 2 litres d'eau glacée et de 100 ml d'acide chlorhydrique à 36%.
Le précipité cristallisé a été essoré, lavé à l'eau, séché et recristallisé de l'acétonitrile au reflux.
On a obtenu 74,2 g de cristaux jaunes de 2-chloro-N-[2-(2-hydroxy-éthylamino)-5-nitro-phényl]-acétamide qui ont fondu à 206°C et dont l'analyse élémentaire calculée pour C₁₀H₁₂N₃O₄Cl était :

| % | C | H | N | O | Cl |
|---|---|---|---|---|---|
| Calculé | 43,89 | 4,42 | 15,35 | 23,38 | 12,95 |
| Trouvé | 43,83 | 4,63 | 15,23 | 22,87 | 13,00 |

### b) Préparation du chlorure de 1-{[2-(2-hydroxy-éthylamino)-5-nitro-phénylcarbamoyl]-méthyl}-3-methyl-3H-imidazol-1-ium

On a chauffé au reflux pendant une heure la suspension de 42,0 g (0,15 mole) de 2-chloro-N-[2-(2-hydroxy-éthylamino)-5-nitro-phényl]-acétamide obtenu ci-dessus à l'étape précédente et de 24,6 g (0,3 mole) de 1-méthyl-1H-imidazole dans 150 ml de toluène.
On a ajouté 30 ml d'isobutanol et prolongé pendant 2 heures le chauffage au reflux.
On a refroidi à température ambiante, essoré, lavé au toluène et recristallisé d'un mélange d'éthanol et d'eau au reflux.
On a obtenu 37,9 g de cristaux jaunes de chlorure de 1-{[2-(2-hydroxyéthylamino)-5-nitro-phénylcarbamoyl]-méthyl}-3-méthyl-3H-imidazol-1-ium qui ont fondu à 200°C et dont l'analyse élémentaire calculée pour C₁₄H₁₈N₅O₄Cl était :

| % | C | H | N | O | Cl |
|---|---|---|---|---|---|
| Calculé | 47,26 | 5,10 | 19,68 | 17,99 | 9,96 |
| Trouvé | 48,04 | 5,20 | 19,87 | 17,03 | 10,28 |

### c) Réduction du chlorure de 1-{[2-(2-hydroxy-éthylamino)-5-nitro-phénylcarbamoyl]-méthyl}-3-méthyl-3H-imidazol-1-ium

On a utilisé le mode opératoire décrit ci-dessus à l'exemple 12, étape b ).
A partir de 37,9 g de chlorure de 1-{[2-(2-hydroxy-éthylamino)-5-nitro-phénylcarbamoyl]-méthyl}-3-méthyl-3H-imidazol-1-ium obtenu à l'étape précédente, on a obtenu 37,1 g de cristaux blancs de chlorure 1-{[5-amino-2-(2-hydroxy-éthylamino)-phénylcarbamoyl]-méthyl}-3-méthyl-3H-imidazol-1-ium, dichlorhydrate qui ont fondu avec décomposition vers 240°C et dont la RMN 1H était conforme à celle du produit attendu.

### EXEMPLES D'APPLICATION

### EXEMPLES 1 à 13 DE TEINTURE EN MILIEU BASIQUE

On a préparé les compositions tinctoriales suivantes (teneurs en grammes) :

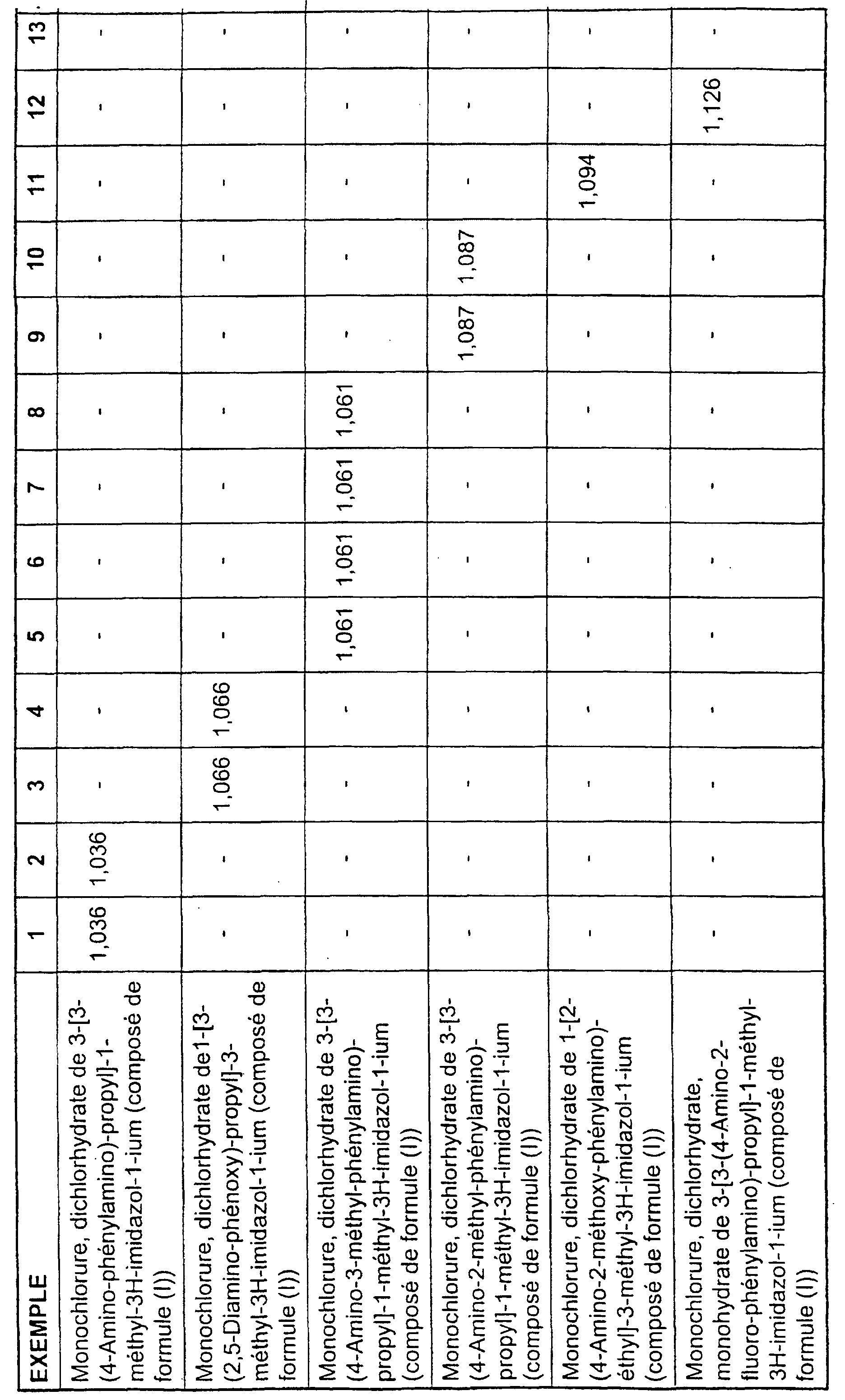

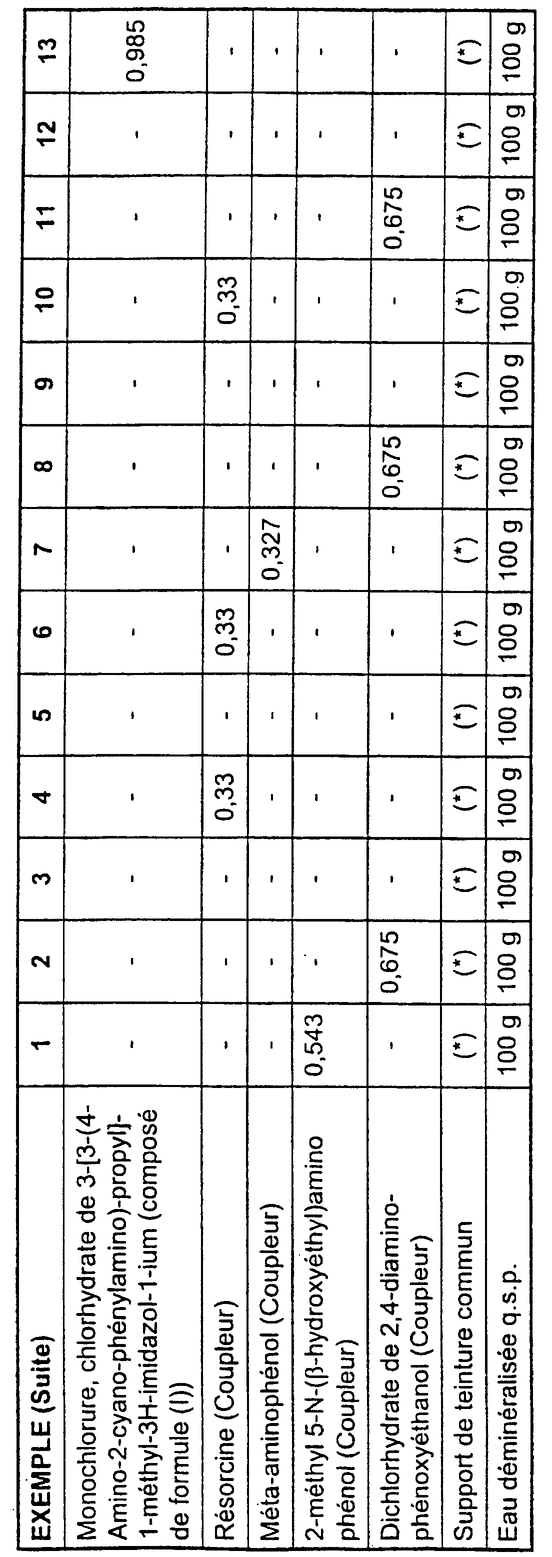

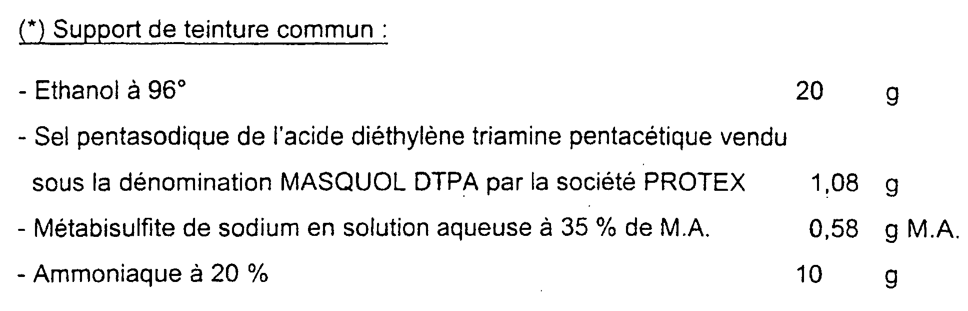

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de TEINTURE** | **Nuance sur cheveux naturels** | **Nuance sur cheveux permanentés** |
|---|---|---|---|
| 1 | 10 ± 0,2 | Violine cendré | Violine profond |
| 2 | 10± 0,2 | Bleu profond | Bleu profond |
| 3 | 10 ± 0,2 | Beige doré | Cendré doré |
| 4 | 10 ± 0,2 | Cendré doré irisé | Naturel violacé |
| 5 | 10 ± 0,2 | Doré mat | Doré mat |
| 6 | 10 ± 0,2 | Cendré doré mat | Cendré doré mat |
| 7 | 10 ± 0,2 | Gris cendré | Gris cendré |
| 8 | 10 ± 0,2 | Bleu vert | Bleu vert |
| 9 | 10 ± 0,2 | Irisé légèrement acajou | Irisé légèrement acajou |
| 10 | 10 ± 0,2 | Cendré acajou | Cendré violacé |
| 11 | 10 ± 0,2 | Gris cendré | Gris cendré |
| 12 | 10 ± 0,2 | Irisé violine | Irisé violine |
| 13 | 10 ± 0,2 | Irisé acajou | Irisé acajou |

### EXEMPLES 14 à 17 DE TEINTURE EN MILIEU BASIQUE

On a préparé les compositions tinctoriales suivantes (teneurs en grammes) :

| **EXEMPLE** | **14** | **15** | **16** | **17** |
|---|---|---|---|---|
| Chlorure de 3-[2-(2,5-diamino-phényl)-éthyl]-1-méthyl-3H-imidazol-1-ium, 2HCl (Composé de formule (I)) | 0,98 | - | - | - |
| Chlorure de 1-{2-[(4-amino-phényl)-éthyl-amino]-éthyl}-3-méthyl-3H-imidazol-1-ium, 2HCl (Composé de formule (I)) | - | 1,06 | - | - |
| Dichlorure de N,N-bis-[2-(3-méthyl-3H-imidazol-1-ium)-éthyl]-4-amino-aniline, monochlorhydrate, monohydrate (Composé de formule (I)) | - | - | 1,41 | - |
| Chlorure de 3-[2-(4-amino-phénylamino)-butyl]-1-méthyl-3H-imidazol-1-ium, dichlorhydrate (Composé de formule (I)) | - | - | - | 1,06 |
| 2,4-diamino 1-(β-hydroxyéthyloxy) benzène, 2HCl (Coupleur) | 0,723 | - | - | - |
| 3-amino phénol (Coupleur) | - | 0,327 | - | - |
| 6-hydroxy indole (Coupleur) | - | - | 0,399 | - |
| 5-N-(β-hydroxyéthyl)amino 2-méthyl phénol (Coupleur) | - | - | - | 0,498 |
| Support de teinture commun | (*) | (*) | (*) | (*) |
| Eau déminéralisée q.s.p. | 100 g | 100 g | 100 g | 100 g |

| | | | | |
|---|---|---|---|---|
| (*) Support de teinture commun : il est identique à celui utilisé pour les exemples de teinture 1 à 13 ci-dessus. | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **14** | 10 ± 0,2 | Bleu violacé |
| **15** | 10 ± 0,2 | Châtain cendré violacé |
| **16** | 10 ± 0,2 | Châtain clair doré cuivré |
| **17** | 10 ± 0,2 | Violine |

## Revendications

1. Composés de formule (I) suivante, et leurs sels d'addition avec un acide : 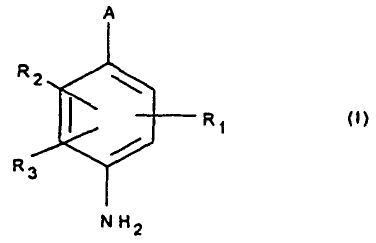 dans laquelle:
• R₁, R₂, R₃, qui peuvent être identiques ou différents, représentent un atome d'hydrogène ; un atome d'halogène ; un groupement Z ; un radical alkyl(C₁-C₆) carbonyle ; un radical carboxy ; un radical alkyl(C₁-C₆)carboxy; un radical carbanyle; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ; ou un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical carboxyalkyle en C₁-C₆; un radical trifluoroalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆) ;
• A représente un groupement -NR₄R₅ ou un radical hydroxyle ;
• R₄ et R₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy (C₁-C₆) alkyle en C₁-C₆ ; un radical trifluoroalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆ ;
• Z est choisi parmi les groupements cationiques insaturés de formule (II) suivante : 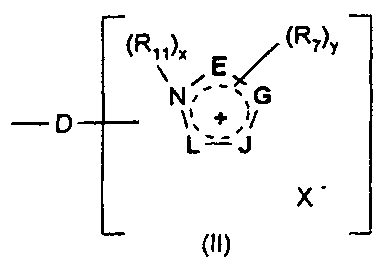
dans lesquelles :
• D est un bras de liaison qui représente une chaîne alkylène comportant de 1 à 14 atomes de carbone, linéaire ou ramifiée.
• les sommets E, G, J, L, identiques ou différents, représentent les atomes de carbone ou d'azote necessaires pour former un cycle pyrolique, pyrazolique, imidazolique ou triazolique.
• R₇ représente un radical alkyle en C₁-C₆, un radical monohydroxyalkyle en C₁-C₆, un radical polyhydroxyalkyle en C₂-C₆ ;
• R₁₁ représente un radical alkyle en C₁-C₆ ; un radical monohydroxyalkyle en C₁-C₆ ; un radical polyhydroxyalkyle en C₂-C₆ ;
• x et y sont des nombres entiers égaux à 0 ou 1 ; avec les conditions suivantes :
- dans les groupements cationiques. insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
• X⁻ représente un anion monovalent ou divalent ;
étant entendu :
que le nombre de groupements cationiques insaturés Z de formule (II) est égal à 1 ;

2. Composés selon la revendication 1, **caractérisés par le fait que** les cycles des groupements insaturés Z de formule (II) sont choisis parmi les cycles pyrrolique, imidazolique, pyrazolique et triazolique.

3. Composés selon la revendication 1 ou 2, **caractérisés par le fait que** les cycles des groupements insaturés Z de formule sont choisis parmi les cycles imidazoliques.

4. Composés selon l'une quelconque des revendications précédentes, **caractérisés par le fait que** X ⁻ est choisi parmi un atome d'halogène, un hydroxyde, un hydrogènesulfate, ou un alkyl(C₁-C₆)sulfate.

5. Composés selon l'une quelconque des revendications précédentes, **caractérisés par le fait qu'**ils sont choisis parmi :
- le bromure de 1-[2-(4-amino-phénylamino)-éthyl]-3-méthyl-3H-imidazol-1-ium ;
- le chlorure de 1-[3-(2,5-diamino-phénoxy)-propyl]-3-méthyl-3H-imidazol-1-ium ;
- le chlorure de 3-[3-(4-amino-phénylamino)-propyl]-1-méthyl-3H-imidazol-1-ium;
- le chlorure de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;
- le chlorure de 3-[3-(4-amino-2-fluoro-phénylamino)-propyl]-1-méthyl-3H-imidazol- 1-ium, monohydrate ;
- le chlorure de 3-[3-(4-amino-2-cyano-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 1-(5-amino-2-hydroxy-benzyl)-3-méthyl-3H-imidazol-1-ium ;
- le chlorure de 1-(5-amino-2-hydroxy-benzyl)-2-méthyl-2H-pyrazol-1-ium ;
- le chlorure de 1-[2-(2,5-diamino-phényl)-éthyl]-3-méthyl-3H-imidazol-1-ium ;
- le chlorure de 3-[2-(2,5-diamino-phényl)-éthyl]-1-méthyl-3H-imidazol-1-ium ;
- le chlorure de 1-{2-[(4-amino-phényl)-éthylamino]-éthyl}-3-méthyl-3H-imidazol-1-ium ;
- le dichlorure de N,N-bis-[2-(3-méthyl-3H-imidazol-1-ium)-éthyl]-4-amino-aniline ;
- le chlorure de 3-[2-(4-amino-phénylamino)-butyl]-1-méthyl-3H-imidazol-1-ium ;
- le bromure de 4-[2-(2,5-diamino-phénoxy)-éthyl]-1,3-diméthyl-3H-imidazol-1-ium ;
- le chlorure de 2-(2,5-diamino-phénoxyméthyl)-1,3-diméthyl-3H-imidazol-1-ium ;
- le chlorure de 4-[3-(4-amino-phénylamino)-propyl]-1,3-diméthyl-3H-imidazol-1-ium ;
- le chlorure de 4-[3-(4-amino-3-méthyl-phénylamino)-propyl]-1,3-diméthyl-3H-imidazol-1-ium ;
- le chlorure de 4-{2-[2-(2-amino-5-hydroxy-phényl)-acétylamino]-éthyl}-1,3-diméthyl-3H-imidazol-1-ium ;
- le chtorure de 4-[(5-amino-2-hydroxy-benzylcarbamoyl)-méthyl]-1,3-diméthyl-3H-imidazol-1-ium ; et leurs sels d'addition avec un acide.

6. Composés choisis parmi :
- le chlorure de 1-{[5-amino-2-(2-hydroxy-éthylamino)-phénylcarbamoyl]-méthyl}-3-méthyl-3H-imidazol-1-ium ;
- le chlorure 4-[(2,5-diamino-phénylcarbamoyl)-méthyl]-1,3-diméthyl-3H-imidazol-1-ium .

7. Utilisation des composés de formule (I) tels que définis à l'une quelconque des revendications précédentes, à titre de base d'oxydation pour la teinture d'oxydation des fibres kératiniques, et en particulier des fibres kératiniques humaines telles que les cheveux.

8. Composition pour la teinture d'oxydation des fibres kératiniques et en particulier des fibres kératiniques humaines telles que les cheveux, **caractérisée par le fait qu'**elle comprend, dans un milieu approprié pour la teinture, au moins un composé de formule (I) tel que défini à l'une quelconque des revendications 1 à 5, ou un composé tel que défini dans la revendication 6, à titre de base d'oxydation.

9. Composition selon la revendication 8, **caractérisée par le fait que** le ou les composés de formule (I) représentent de 0,0005 à 12 % en poids du poids total de la composition tinctoriale.

10. Composition selon la revendication 9, **caractérisée par le fait que** le ou les composés de formule (I) représentent de 0,005 à 6 % en poids du poids total de la composition tinctoriale.

11. Composition selon l'une quelconque des revendications 8 à 10, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

12. Composition selon l'une quelconque des revendications 8 à 11, **caractérisée par le fait qu'**elle présente un pH compris entre 3 et 12.

13. Composition selon l'une quelconque des revendications 8 à 12, **caractérisée par le fait qu'**elle renferme au moins une base d'oxydation additionnelle choisie parmi les paraphénylènediamines différentes des composés de formule (I), les bis-phénylalkylènediamines, les para-aminophénols différents des composés de formule (I), les ortho-aminophénols et les bases hétérocycliques.

14. Composition selon la revendication 13, **caractérisée par le fait que** la ou les bases d'oxydation additionnelles représentent de 0,0005 à 12 % en poids du poids total de la composition tinctoriale.

15. Composition selon l'une quelconque des revendications 8 à 14, **caractérisée par le fait qu'**elle renferme au moins un coupleur et/ou au moins un colorant direct.

16. Composition selon la revendication 15, **caractérisée par le fait que** le ou les coupleurs sont choisis parmi les métaphénylènediamines, les méta-aminophénols, les métadiphénols et les coupleurs hétérocycliques, et leurs sels d'addition avec un acide.

17. Composition selon la revendication 15 ou 16, **caractérisée par le fait que** le ou les coupleurs représentent de 0,0001 à 10 % en poids du poids total de la composition tinctoriale.

18. Composition selon l'une quelconque des revendications 8 à 17, **caractérisée par le fait que** les sels d'addition avec un acide sont choisis parmi les chlorhydrates, les bromhydrates, les sulfates, les citrates, les succinates, les tartrates, les lactates et les acétates.

19. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 8 à 18, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

20. Procédé selon la revendication 19, **caractérisé par le fait que** l'agent oxydant est choisi parmi le peroxyde d'hydrogène, le peroxyde d'urée, les bromates de métaux alcalins, les persels tels que les perborates et persulfates.

21. Dispositif à plusieurs compartiments, ou "kit" de teinture à plusieurs compartiments, dont un premier compartiment renferme une composition tinctoriale telle que définie à l'une quelconque des revendications 8 à 18 et un second compartiment renferme une composition oxydante.

## Patentansprüche

1. Verbindungen der folgenden Formel (I) und ihre Additionssalze mit einer Säure: 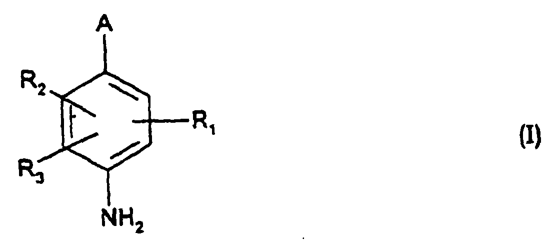 wobei in der Formel (I):
· die Gruppen R₁, R₂ und R₃, die identisch oder voneinander verschieden sein können, bedeuten Wasserstoff; Halogen; eine Gruppe Z; Alkyl(C₁₋₆)carbonyl; Carboxy; Alkyl(C₁₋₆)carboxyl; Carbamoyl; C₁₋₆-Alkyl; C₁₋₆-Monohydroxyalkyl; C₂₋₆-Polyhydroxyalkyl; Alkoxy(C₁₋₆)alkyl(C₁₋₆); C₁₋₆-Trifluoralkyl; Cyano; OR₆; oder C₁₋₆-Aminoalkyl, worin die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl;
· die Gruppe R₆ bedeutet C₁₋₆-Alkyl; C₁₋₆-Monohydroxyalkyl; C₂₋₆-Polyhydroxyalkyl; eine Gruppe Z; Alkoxy(C₁₋₆)alkyl(C₁₋₆); C₁₋₆-Carboxyalkyl; C₁₋₆-Trifluoralkyl; C₁₋₆-Aminoalkyl; C₁₋₆-Aminoalkyl, worin die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl;
· die Gruppe A bedeutet eine Gruppe -NR₄R₅ oder die Hydroxygruppe;
· die Gruppen R₄ und R₅, die identisch oder voneinander verschieden sind, bedeuten Wasserstoff; eine Gruppe Z; C₁₋₆-Alkyl; C₁₋₆-Monohydroxyalkyl; C₂₋₆-Polyhydroxyalkyl; Alkoxy-(C₁₋₆)alkyl(C₁₋₆); C₁₋₆-Trifluoralkyl; C₁₋₆-Aminoalkyl; C₁₋₆-Aminoalkyl, wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl;
· Z ist unter den ungesättigten kationischen Gruppen der folgenden Formel (II) ausgewählt: 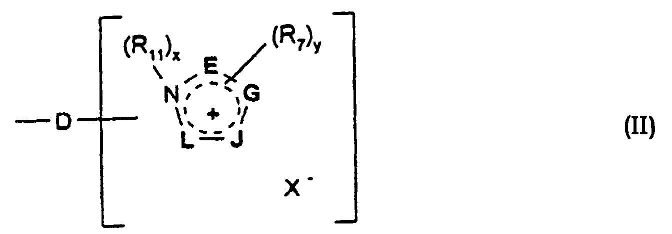
worin bedeuten:
· D eine Verbindungsgruppe, bei der es sich um eine geradkettige oder verzweigte Alkylenkette mit 1 bis 14 Kohlenstoffatome handelt;
· die Atome E, G, J und L, die gleich oder verschieden sind, bedeuten die zur Bildung eines Pyrrolrings, Imidazolrings, Pyrazolrings oder Triazolrings notwendigen Kohlenstoffatome oder Stickstoffatome;
· R₇ bedeutet C₁₋₆-Alkyl, C₁₋₆-Monohydroxyalkyl oder C₂₋₆-Polyhydroxyalkyl;
· R₁₁ bedeutet C₁₋₆-Alkyl, C₁₋₆-Monohydroxyalkyl oder C₂₋₆-Polyhydroxyalkyl;
· x und y bedeuten 0 oder 1; mit den folgenden Bedingungen:
- in den ungesättigten kationischen Gruppen der Formel (II):
- die Gruppe D ist an das Stickstoffatom gebunden, wenn x = 0,
- die Gruppe D ist an E, G, J oder L gebunden, wenn x = 1,
- y kann nur den Wert 1 annehmen, wenn:
1) wenn die Atome E, G, J und L alle ein Kohlenstoffatom bedeuten, falls R₇ von dem Stickstoffatom des ungesättigten Rings getragen wird;
2) wenn mindestens ein Atom E, G, J oder L ein Stickstoffatom bedeutet, an das R₇ gebunden ist;
· X⁻ bedeutet ein einwertiges oder zweiwertiges Anion;
mit der Maßgabe, dass:
die Anzahl der kationischen Gruppen Z der Formel (II) 1 ist.

2. Verbindungen nach Anspruch 1, **dadurch gekennzeichnet, dass** die Ringe der ungesättigten Gruppen Z der Formel (II) unter den Ringen Pyrrol, Imidazol, Pyrazol und Triazol ausgewählt sind.

3. Verbindungen nach Anspruch 1 oder 2, **dadurch gekennzeichnet, dass** die Ringe der ungesättigten Gruppen Z der Formel (II) unter den Imidazolringen ausgewählt sind.

4. Verbindungen nach einem der vorhergehenden Ansprüche, **dadurch gekennzeichnet, dass** X⁻ unter Halogen, Hydroxid, Hydrogensulfat oder C₁₋₆-Alkylsulfat ausgewählt ist.

5. Verbindungen nach einem der vorhergehenden Ansprüche, **dadurch gekennzeichnet, dass** sie unter den folgenden Verbindungen ausgewählt sind:
- 1-[2-(4-Amino-phenylamino)-ethyl]-3-methyl-3H-imidazol-1-ium-bromid;
- 1-[3-(2,5-Diamino-phenoxy)-propyl]-3-methyl-3H-imidazol-1-ium-chlorid;
- 3-[3-(4-Amino-phenyl)-propylamino]-1-methyl-3H-imidazol-1-ium-chlorid;
- 3-[3-(4-Amino-3-methyl-phenylamino)-propyl]-1-methyl-3Himidazol-1-ium-chlorid;
- 3-[3-(4-Amino-2-methyl-phenylamino)-propyl]-1-methyl-3Himidazol-1-ium-chlorid;
- 3-[3-(4-Amino-2-fluor-phenylamino)-propyl]-1-methyl-3Himidazol-1-ium-chlorid-Monohydrat;
- 3-[3-(4-Amino-2-cyano-phenylamino)-propyl]-1-methyl-3Himidazol-1-ium-chlorid;
- 1-[2-(4-Amino-2-methoxy-phenylamino)-ethyl]-3-methyl-3Himidazol-1-ium-chlorid;
- 1-(5-Amino-2-hydroxy-benzyl)-3-methyl-3H-imidazol-1-iumchlorid;
- 1-(5-Amino-2-hydroxy-benzyl)-2-methyl-2H-pyrazol-1-iumchlorid;
- 1-[2-(2,5-Diamino-phenyl)-ethyl]-3-methyl-3H-imidazol-1-ium-chlorid;
- 3-[2-(2,5-Diamino-phenyl)-ethyl]-1-methyl-3H-imidazol-1-ium-chlorid;
- 1-{2-[(4-Amino-phenyl)-ethyl-amino]-ethyl}-3-methyl-3Himidazol-1-ium-chlorid;
- N,N-Bis[2-(3-methyl-3H-imidazol-1-ium)-ethyl]-4-aminoanilin;
- 3-[2-(4-Amino-phenylamino)-butyl]-1-methyl-3H-imidazol-1-ium-chlorid;
- 4-[2-(2,5-Diamino-phenoxy)-ethyl]-1,3-dimethyl-3H-imidazol-1-ium-bromid;
- 2-(2,5-Diamino-phenoxymethyl)-1,3-dimethyl-3H-imidazol-1-ium-chlorid;
- 4-[3-(4-Amino-phenylamino)-propyl]-1,3-dimethyl-3Himidazol-1-ium-chlorid;
- 4-[3-(4-Amino-3-methyl-phenylamino)-propyl]-1,3-dimethyl-3H-imidazol-1-ium-chlorid;
- 4-{2-[2-(2-Amino-5-hydroxy-phenyl)-acetylamino]-ethyl}-1,3-dimethyl-3H-imidazol-1-ium-chlorid;
- 4-[(5-Amino-2-hydroxy-benzylcarbamoyl)-methyl]-1,3-dimethyl-3H-imidazol-1-ium-chlorid; und
- den Additionssalzen dieser Verbindungen mit einer Säure.

6. Verbindungen, die ausgewählt sind unter:
· 1-{[5-Amino-2-(2-hydroxy-ethylamino)-phenylcarbamoyl]-methyl}-3-methyl-3H-imidazol-1-ium-chlorid; und
· 4-[(2,5-Diamino-phenylcarbamoyl]-methyl}-1,3-dimethyl-3Himidazol- 1-ium-chlohd.

7. Verwendung von Verbindungen der Formel (I) nach einem der vorhergehenden Ansprüche als Oxidationsbasen zum oxidativen Färben von Keratinfasern und insbesondere menschlichen Keratinfasern, wie dem Haar.

8. Zusammensetzung zum oxidativen Färben von Keratinfasern und insbesondere menschlichen Keratinfasern, wie dem Haar, **dadurch gekennzeichnet, dass** sie in einem zum Färben geeigneten Medium als Oxidationsbase mindestens eine Verbindung der Formel (I) nach einem der Ansprüche 1 bis 5 oder eine Verbindung gemäß Anspruch 6 enthält.

9. Zusammensetzung nach Anspruch 8, **dadurch gekennzeichnet, dass** die Verbindung(en) der Formel (I) 0,0005 bis 12 Gew.-% des Gesamtgewichts der Farbmittelzusammensetzung ausmachen.

10. Zusammensetzung nach Anspruch 9, **dadurch gekennzeichnet, dass** die Verbindung(en) der Formel (I) 0,005 bis 6 Gew.-% des Gesamtgewichts der Farbmittelzusammensetzung ausmachen.

11. Zusammensetzung nach einem der Ansprüche 8 bis 10, **dadurch gekennzeichnet, dass** das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen, analogen Produkten und deren Gemischen ausgewählt ist.

12. Zusammensetzung nach einem der Ansprüche 8 bis 11, **dadurch gekennzeichnet, dass** sie einen pH-Wert im Bereich von 3 bis 12 aufweist.

13. Zusammensetzung nach einem der Ansprüche 8 bis 12, **dadurch gekennzeichnet, dass** sie mindestens eine zusätzliche Oxidationsbase enthält, die unter den p-Phenylendiaminen, die von den Verbindungen der Formel (I) verschieden sind, den Bisphenylalkylendiaminen, den p-Aminophenolen, die von den Verbindungen der Formel (I) verschieden sind, den o-Aminophenolen und den heterocyclischen Basen ausgewählt ist.

14. Zusammensetzung nach Anspruch 13, **dadurch gekennzeichnet, dass** die zusätzliche(n) Oxidationsbase(n) 0,0005 bis 12 Gew.-% des Gesamtgewichts der Farbmittelzusammensetzung ausmachen.

15. Zusammensetzung nach einem der Ansprüche 8 bis 14, **dadurch gekennzeichnet, dass** sie mindestens einen Kuppler und/oder mindestens einen Direktfarbstoff enthält.

16. Zusammensetzung nach Anspruch 15, **dadurch gekennzeichnet, dass** der oder die Kuppler unter den m-Phenylendiaminen, m-Aminophenolen, m-Dihydroxybenzolen und den heterocyclischen Kupplern und deren Additionssalzen mit einer Säure ausgewählt sind.

17. Zusammensetzung nach Anspruch 15 oder 16, **dadurch gekennzeichnet, dass** der oder die Kuppler 0,0001 bis 10 Gew.-% des Gesamtgewichts der Farbmittelzusammensetzung ausmachen.

18. Zusammensetzung nach einem der Ansprüche 8 bis 17, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

19. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 8 bis 18 aufgebracht wird, wobei die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

20. Verfahren nach Anspruch 19, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, ausgewählt ist.

21. Vorrichtung mit mehreren Abteilungen oder "Kit" zum Färben mit mehreren Abteilungen, wobei eine erste Abteilung eine Farbmittelzusammensetzung nach einem der Ansprüche 8 bis 18 und eine zweite Abteilung eine oxidierende Zusammensetzung enthält.

## Claims

1. Compounds of formula (I) below, and the addition salts thereof with an acid: 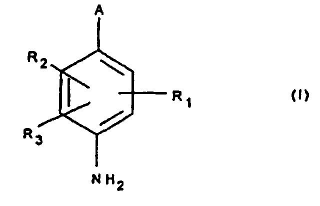 in which:
• R₁, R₂ and R₃, which may be identical or different, represent a hydrogen atom; a halogen atom; a group Z; a (C₁-C₆)alkylcarbonyl radical; a carboxyl radical; a (C₁-C₆) alkylcarboxyl radical; a carbamyl radical; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a cyano radical; a group OR₆; or a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl radicals;
• R₆ denotes a C₁-C₆ alkyl radical; a C₁-C₆ monohydroxyalkyl radical; a C₂-C₆ polyhydroxyalkyl radical; a group Z; a (C₁-C₆)alkoxy(C₁-C₆)alkyl radical; a carboxy(C₁-C₆)alkyl radical ; a C₁-C₆ trifluoroalkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl radicals;
• A represents a group -NR₄R₅ or a hydroxyl radical;
• R₄ and R₅, which may be identical or different, represent a hydrogen atom; a group Z; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a C₁-C₆ amino alkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl radicals;
• Z is chosen from the unsaturated cationic groups of formula (II) below: 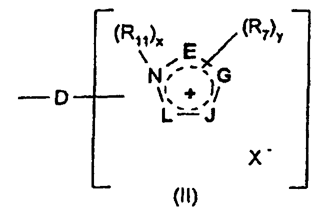
in which:
• D is a linker arm which represents a linear or branched alkyl chain containing from 1 to 14 carbon atoms;
• the ring members E, G, J, L, which may be identical or different, represent the carbon or nitrogen atoms which are necessary to form a pyrrole, pyrazole, imidazole or triazole ring;
• R₇ represents a C₁-C₆ alkyl radical, a C₁-C₆ monohydroxyalkyl radical, a C₂-C₆ polyhydroxyalkyl radical;
• R₁₁ represents a C₁-C₆ alkyl radical; a C₁-C₆ monohydroxyalkyl radical; a C₂-C₆ polyhydroxyalkyl radical;
• x and y are integers equal to 0 or 1; with the following conditions:
- in the unsaturated cationic groups of formula (II):
- when x = 0, the linker arm D is attached to the nitrogen atom,
- when x = 1, the linker arm D is attached to one of the ring members E, G, J or L,
- y can take the value 1 only:
1) when the ring members E, G, J and L simultaneously represent a carbon atom and when the radical R₇ is borne by the nitrogen atom of the unsaturated ring; or alternatively
2) when at least one of the ring members E, G, J and L represents a nitrogen atom to which the radical R₇ is attached;
• X⁻ represents a monovalent or divalent anion;
it being understood:
that the number of unsaturated cationic groups Z of formula (II) is equal to 1.

2. Compounds according to Claim 1, **characterized in that** the rings of the unsaturated groups Z of formula (II) are chosen from pyrrole, imidazole, pyrazole, and triazole rings.

3. Compounds according to Claim 1 or 2, **characterized in that** the rings of the unsaturated groups Z of formula (II) are chosen from imidazole rings.

4. Compounds according to any one of the preceding claims, **characterized in that** X⁻ is chosen from a halogen atom, a hydroxide, a hydrogensulphate and a C₁-C₆ alkyl sulphate.

5. Compounds according to any one of the preceding claims, **characterized in that** they are chosen from:
- 1-[2-(4-aminophenylamino)ethyl]-3-methyl-3H-imidazol-1-ium bromide;
- 1-[3-(2,5-diaminophenoxy)propyl]-3-methyl-3H-imidazol-1-ium chloride;
- 3-[3-(4-aminophenylamino)propyl]-1-methyl-3H-imidazol-1-ium chloride;
- 3-[3-(4-amino-3-methylphenylamino)propyl]-1-methyl-3H-imidazol-1-ium chloride;
- 3-[3-(4-amino-2-methylphenylamino)propyl]-1-methyl-3H-imidazol-1-ium chloride;
- 3-[3-(4-amino-2-fluorophenylamino)propyl]-1-methyl-3H-imidazol-1-ium chloride monohydrate;
- 3-[3-(4-amino-2-cyanophenylamino)propyl]-1-methyl-3H-imidazol-1-ium chloride;
- 1-[2-(4-amino-2-methoxyphenylamino)ethyl]-3-methyl-3H-imidazol-1-ium chloride;
- 1-(5-amino-2-hydroxybenzyl)-3-methyl-3H-imidazol-1-ium chloride;
- 1-(5-amino-2-hydroxybenzyl)-2-methyl-2H-pyrazol-1-ium chloride;
- 1-[2-(2,5-diaminophenyl)ethyl]-3-methyl-3H-imidazol-1-ium chloride;
- 3-[2-(2,5-diaminophenyl)ethyl]-1-methyl-3H-imidazol-1-ium chloride;
- 1-{2-[(4-aminophenyl)ethylamino]ethyl}-3-methyl-3H-imidazol-1-ium chloride;
- N,N-bis[2-(3-methyl-3H-imidazol-1-ium)ethyl]-4-aminoaniline dichloride;
- 3-[2-(4-aminophenylamino)butyl]-1-methyl-3H-imidazol-1-ium chloride;
- 4-[2-(2,5-diaminophenoxy)ethyl]-1,3-dimethyl-3H-imidazol-1-ium bromide;
- 2-(2,5-diaminophenoxymethyl)-1,3-dimethyl-3H-imidazol-1-ium chloride;
- 4-[3-(4-aminophenylamino)propyl]-1,3-dimethyl-3H-imidazol-1-ium chloride;
- 4-[3-(4-amino-3-methylphenylamino)propyl]-1,3-dimethyl-3H-imidazol-1-ium chloride;
- 4-{2-[2-(2-amino-5-hydroxyphenyl)acetylamino]ethyl}-1,3-dimethyl-3H-imidazol-1-ium chloride;
- 4-[(5-amino-2-hydroxybenzylcarbamoyl)methyl]-1,3-dimethyl-3H-imidazol-1-ium chloride;
and the addition salts thereof with an acid.

6. Compounds chosen from:
- 1-{[5-amino-2-(2-hydroxyethylamino)phenylcarbamoyl]-methyl}-3-methyl-3H-imidazol-1-ium chloride;
- 4-[(2,5-diaminophenylcarbamoyl)methyl]-1,3-dimethyl-3H-imidazol-1-ium chloride.

7. Use of the compounds of formula (I) as defined in any one of the preceding claims, as oxidation bases for the oxidation dyeing of keratin fibres, and in particular of human keratin fibres such as the hair.

8. Composition for the oxidation dyeing of keratin fibres, and in particular of human keratin fibres such as the hair, **characterized in that** it comprises, in a medium which is suitable for dyeing, at least one compound of formula (I) as defined in any one of Claims 1 to 5, or a compound as defined in Claim 6, as an oxidation base.

9. Composition according to Claim 8, **characterized in that** the compound(s) of formula (I) represent(s) from 0.0005 to 12% by weight relative to the total weight of the dye composition.

10. Composition according to Claim 9, **characterized in that** the compound(s) of formula (I) represent(s) from 0.005 to 6% by weight relative to the total weight of the dye composition.

11. Composition according to any one of Claims 8 to 10, **characterized in that** the medium which is suitable for dyeing (or the support) consists of water or a mixture of water and at least one organic solvent chosen from C₁-C₄ lower alkanols, glycerol, glycols and glycol ethers, aromatic alcohols, similar products and mixtures thereof.

12. Composition according to any one of Claims 8 to 11, **characterized in that** it has a pH of between 3 and 12.

13. Composition according to any one of Claims 8 to 12, **characterized in that** it contains at least one additional oxidation base chosen from paraphenylenediamines other than the compounds of formula (I), bis(phenyl)alkylenediamines, para-aminophenols other than the compounds of formula (I), orthoaminophenols and heterocyclic bases.

14. Composition according to Claim 13, **characterized in that** the additional oxidation base(s) represent(s) from 0.0005 to 12% by weight relative to the total weight of the dye composition.

15. Composition according to any one of Claims 8 to 14, **characterized in that** it contains at least one coupler and/or at least one direct dye.

16. Composition according to Claim 15, **characterized in that** the coupler(s) is (are) chosen from meta-phenylenediamines, meta-aminophenols, meta-diphenols and heterocyclic couplers, and the addition salts thereof with an acid.

17. Composition according to Claim 15 or 16, **characterized in that** the coupler(s) represent(s) from 0.0001 to 10% by weight relative to the total weight of the dye composition.

18. Composition according to any one of Claims 8 to 17, **characterized in that** the addition salts with an acid are chosen from the hydrochlorides, hydrobromides, sulphates, citrates, succinates, tartrates, lactates and acetates.

19. Process for dyeing keratin fibres, and in particular human keratin fibres such as the hair, **characterized in that** at least one dye composition as defined in any one of Claims 8 to 18 is applied to these fibres and the colour is developed at acidic, neutral or alkaline pH using an oxidizing agent which is added to the dye composition just at the time of use, or which is present in an oxidizing composition which is applied simultaneously or sequentially in a separate manner.

20. Process according to Claim 19, **characterized in that** the oxidizing agent is chosen from hydrogen peroxide, urea peroxide, alkali metal bromates and persalts such as perborates and persulphates.

21. Multi-compartment dyeing device or multicompartment dyeing kit, a first compartment of which contains a dye composition as defined in any one of Claims 8 to 18, and a second compartment of which contains an oxidizing composition.
